# EUROPEAN PATENT APPLICATION

(11) **EP 2 175 266 A1**
(43) Date of publication of application: **14.04.2010**
(21) Application number: 08791529.4
(22) Date of filing: 24.07.2008
(51) Int. Cl.: G01N 27/62, C12M 1/00, C12Q 1/68

(54) **APPARATUS, PROCESS AND PROGRAM FOR ANALYZING NUCLEIC ACID BASE SEQUENCE AND BASE MODIFICATION**

(30) Priority: 27.07.2007 JP 2007195555
(71) Applicant: The University of Tokyo, Bunkyo-Ku Tokyo 113-8654 (JP); Japan Biological Informatics Consortium, Koto-ku Tokyo 135-8073 (JP)
(72) Inventor: SUZUKI, Tsutomu, Tokyo 113-8654 (JP); UEDA, Hiroki, Tokyo 135-8073 (JP)
(74) Representative: Winter, Brandl, Fürniss, Hübner Röss, Kaiser, Polte Partnerschaft Patent- und Rechtsanwaltskanzlei
(86) International application number: PCT/JP2008/063277
(87) International publication number: WO 2009/017023

(57) **Abstract**

Provided is an apparatus by which a nucleic acid may be analyzed quickly and precisely. Specifically, provided is an apparatus for analysis of a nucleic acid including: a processing unit for creating, based on a total base composition of a nucleic acid, a plurality of base composition sets, creating a hierarchical structure in which the base composition sets are hierarchized in ascending order of a number of bases in a partial base composition at a terminal position, and further creating connection relations between each of the base composition sets, and another base composition set having, at the terminal position, a partial base composition obtained by adding one base to a partial base composition at the terminal position of each of the base composition sets; a processing unit for calculating, for each of the partial base compositions, a predicted mass value of a corresponding product ion, and imparting a weight to each of the base composition sets based on a comparison between the predicted mass value and an actual mass value of a product ion derived from the nucleic acid; and a processing unit for selecting one of base composition sets belonging to each hierarchy while following the connection relations from an outermost hierarchy in the hierarchical structure, based on a weight imparted to each of the base composition sets, to thereby determine a base sequence sequentially from a terminal base of the nucleic acid.

## Description

### TECHNICAL FIELD

The present invention relates to an apparatus, a method, and a program for analysis of a base sequence and a base modification of a nucleic acid, in particular, an apparatus, a method, and a program for analysis of a base sequence and a base modification of a nucleic acid by utilizing mass spectrometry results.

### BACKGROUND ART

Through the discovery of RNA interference and micro-RNA, novel functions of RNA (functional RNA) which does not encode any protein have attracted attention. It has been gradually revealed that functional RNA is a final gene product in itself, behaves as a functional polymer, and plays important roles involved in higher order biological phenomena such as gene expression modulation, development, and differentiation. Further, there is a reported case where the abnormality in functional RNA causes diseases. Thus, there has been pointed out a need for taking into consideration, as a cause of diseases, the abnormality in RNA as well as the abnormality in protein.

Conventionally, a method involving amplifying cDNA by reverse transcription PCR and determining a sequence of the cDNA has been used as a method for analysis of such RNA, for example.

Further, for example, Non-patent Document 1 proposes a method involving creating all of base sequence candidates to be expected as a base sequence of a nucleic acid as an analytic target, scoring each of all of the base sequence candidates by comparing mass/charge ratios theoretically predicted for a plurality of sets of product ions which may be produced from each of all of the base sequence candidates with mass/charge ratios obtained by actual mass spectrometry for product ions produced from the nucleic acid, and determining a candidate having a high score as a base sequence of the nucleic acid.
Non-patent Document 1: Oberacher H. , Mayr B. M., Huber C. G. ; Journal of the American Society for Mass Spectrometry, 15(1), pp. 32-42, 2004.

### DISCLOSURE OF THE INVENTION

### Problems to be Solved by the Invention

However, the above-mentioned method using cDNA requires a skilled technique, takes much time and effort, and besides, is far from being quantitative in view of a bias due to PCR.

Further, the method described in Non-patent Document 1 above requires an enormous amount of calculation in determining a base sequence because the number of expected base sequence candidates is drastically increased along with increasing number of bases in a nucleic acid of interest. Further, the method scores each of base sequence candidates based on the results obtained by comparing a theoretical value with an observed value for all of a plurality of sets of product ions which may be produced from each of the base sequence candidates. Therefore, for example, the method may not take sufficient advantage of highly reliable observed results of a short product ion derived from the terminus. The analysis of a base modification of a nucleic acid has also had a similar problem.

The present invention has been made in view of the above-mentioned problems. One of the objects of the present invention is to provide an apparatus, a method, and a program by which a nucleic acid may be analyzed quickly and exactly for its base sequence, base modification, and the like.

### Means for Solving the Problems

An apparatus for analysis of a nucleic acid according to one embodiment of the present invention for solving the above-mentioned problems is characterized by including: a first processing unit for preparing, based on a total base composition of a nucleic acid, a plurality of base composition sets each formed of a set of partial base compositions, forming a hierarchical structure in which the base composition sets are hierarchized in ascending order of a number of bases in a partial base composition at at least one terminal position thereof, and further forming connection relations between each of the base composition sets, and another base composition set having, at the terminal position, a partial base composition obtained by adding one base to a partial base composition at the terminal position of each of the base composition sets; a second processing unit for calculating, for each of the partial base compositions, a predicted mass value in mass spectrometry of a corresponding product ion, and imparting a weight to each of the base composition sets based on a comparison between the predicted mass value and an actual mass value actually obtained in mass spectrometry of a product ion derived from the nucleic acid; and a third processing unit for performing selection processing for selecting one of base composition sets belonging to each hierarchy while following the connection relations from an outermost hierarchy to which the base composition sets each having a smallest number of bases in a partial base composition at the terminal position belong in the hierarchical structure, based on a weight imparted to each of the base composition sets, to thereby determine a base sequence sequentially from at least one terminus corresponding to the terminal position of the nucleic acid. According to the present invention, there may be provided an apparatus with which a nucleic acid may be analyzed quickly and precisely for its base sequence, base modification, and the like. That is, an analysis with high reliability may be performed for, for example, the determination of a base sequence, the presence or absence of a base modification, and the content of a base modification (e.g., the kind, the position, and the number of the modification) by effectively utilizing mass spectrometry results of a product ion derived from the terminus while effectively reducing an amount of calculation.

In addition, the first processing unit may also hierarchize the base composition sets in ascending order of a number of bases in a partial base composition at one terminal position of a 5'-terminal position and a 3'-terminal position from one outermost hierarchy in the hierarchical structure, and in ascending order of a number of bases in a partial base composition at the other terminal position of a 5'-terminal position and a 3'-terminal position from the other outermost hierarchy, and the third processing unit may also perform the selection processing while following the connection relations from both outermost hierarchies of the one outermost hierarchy and the other outermost hierarchy in the hierarchical structure to internal hierarchies, to thereby determine a base sequence sequentially from both termini of a 5'-terminus and a 3'-terminus of the nucleic acid. In addition, the second processing unit may also impart a weight to each of the connection relations based on a weight imparted to a pair of the base composition sets to be connected with each of the connection relations, and the third processing unit may also perform the selection processing based on a weight imparted to each of the base composition sets and a weight imparted to each of the connection relations. In addition, the first processing unit may also create a directed graph having hierarchized nodes corresponding to each of the base composition sets, and edges for connecting the nodes in a corresponding manner to each of the connection relations, and the third processing unit may also perform the selection processing by searching an optimum route from a terminus on a side of a hierarchy to which the base composition sets each having a smallest number of bases in a partial base composition at the terminal position belong in the directed graph, based on a weight imparted to each of the base composition sets. In this case, the third processing unit may also perform the selection processing by applying dynamic programming. Further, in this case, the first processing unit may hierarchize the nodes in ascending order of a number of bases in a partial base composition at one terminal position of a 5'-terminal position and a 3'-terminal position of the corresponding base composition sets from one terminus in the directed graph, and in ascending order of a number of bases in a partial base composition at the other terminal position of a 5'-terminal position and a 3'-terminal position of the corresponding base composition sets from the other terminus of the directed graph, and the third processing unit may perform the selection processing while following the edges by applying dynamic programming inward from both termini of the one terminus and the other terminus in the directed graph, to thereby determine a base sequence sequentially from both termini of a 5'-terminus and a 3'-terminus of the nucleic acid.

In addition, the second processing unit may also create, for each of the partial base compositions, a first predicted mass value to be predicted when an assumption is made that no modified base is contained in the corresponding product ion, and a second predicted mass value to be predicted when an assumption is made that a modified base is contained in the corresponding product ion in mass spectrometry of the corresponding product ion, and impart a weight to each of the partial base compositions based on a comparison of each of the first predicted mass value and the second predicted mass value with the actual mass value. In this case, the second processing unit may also judge whether or not the modified base is contained in the corresponding product ion for each of the partial base compositions, based on a comparison of each of the first predicted mass value and the second predicted mass value with the actual mass value, and hold each of the partial base composition linked to modification information relating to the modified base, provided that the second processing unit judges that the modified base is contained in the corresponding product ion, and the third processing unit may also determine a position of a modified base contained in the nucleic acid based on judgment results of whether or not a position of a partial base composition linked to the modification information moves in the selected base composition set between adjacent hierarchies in the hierarchical structure.

A method for analysis of a nucleic acid according to one embodiment of the present invention for solving the above-mentioned problems is characterized by including: a first step for creating, based on a total base composition of a nucleic acid, a plurality of base composition sets each consisting of a set of partial base compositions, creating a hierarchical structure in which the base composition sets are hierarchized in ascending order of a number of bases in a partial base composition at at least one terminal position thereof, and further creating connection relations between each of the base composition sets, and another base composition set having, at the terminal position, a partial base composition obtained by adding one base to a partial base composition at the terminal position of each of the base composition sets; a second step for calculating, for each of the partial base compositions, a predictedmass value inmass spectrometry of a corresponding product ion, and imparting a weight to each of the base composition sets based on a comparison between the predicted mass value and an actual mass value actually obtained in mass spectrometry of a product ion derived from the nucleic acid; and a third step for performing selection processing for selecting one of base composition sets belonging to each hierarchy while following the connection relations from an outermost hierarchy to which the base composition sets each having a smallest number of bases in a partial base composition at the terminal position belong in the hierarchical structure, based on a weight imparted to each of the base composition sets, to thereby determine a base sequence sequentially from at least one terminus corresponding to the terminal position of the nucleic acid. According to the present invention, there may be provided a method by which a nucleic acid may be analyzed quickly and precisely.

A program for analysis of a nucleic acid according to one embodiment of the present invention for solving the above-mentioned problems is characterized by making a computer carry out: a first procedure for creating, based on a total base composition of a nucleic acid, a plurality of base composition sets each consisting of a set of partial base compositions, creating a hierarchical structure in which the base composition sets are hierarchized in ascending order of a number of bases in a partial base composition at at least one terminal position thereof, and further creating connection relations between each of the base composition sets, and another base composition set having, at the terminal position, a partial base composition obtained by adding one base to a partial base composition at the terminal position of each of the base composition sets; a second procedure for calculating, for each of the partial base compositions, a predicted mass value in mass spectrometry of a corresponding product ion, and imparting a weight to each of the base composition sets based on a comparison between the predicted mass value and an actual mass value actually obtained in mass spectrometry of a product ion derived from the nucleic acid; and a third procedure for performing selection processing for selecting one of base composition sets belonging to each hierarchy while following the connection relations from an outermost hierarchy to which the base composition sets each having a smallest number of bases in a partial base composition at the terminal position belong in the hierarchical structure, based on a weight imparted to each of the base composition sets, to thereby determine a base sequence sequentially from at least one terminus corresponding to the terminal position of the nucleic acid. According to the present invention, there may be provided a program with which a nucleic acid may be analyzed quickly and exactly.

### BRIEF DESCRIPTION OF THE DRAWINGS

FIG. 1 is a block diagram illustrating a main structure of an apparatus for analysis of a nucleic acid according to one embodiment of the present invention.
FIG. 2 is a function block diagram illustrating main processing performed by a control unit in an apparatus for analysis of a nucleic acid according to one embodiment of the present invention.
FIG. 3 is a flow diagram illustrating main processing included in a method for analysis of a nucleic acid according to one embodiment of the present invention.
FIG. 4 is an explanatory diagram illustrating an example of hierarchy data to be created in one embodiment of the present invention.
FIG. 5 is an explanatory diagram illustrating an example of connection data to be created in one embodiment of the present invention.
FIG. 6 is an explanatory diagram illustrating an example of a directed graph to be created in one embodiment of the present invention.
FIG. 7 is an explanatory diagram of creation of a theoretical spectrum in one embodiment of the present invention.
FIG. 8 is an explanatory diagram of an example of matching processing in one embodiment of the present invention.
FIG. 9 is an explanatory diagram of an example of weighting processing in a directed graph to be created in one embodiment of the present invention.
FIG. 10 is an explanatory diagram of an example of trace back processing in a directed graph to be created in one embodiment of the present invention.
FIG. 11 is an explanatory diagram of an example of base sequence determination using a directed graph to be created in one embodiment of the present invention.
FIG. 12 is an explanatory diagram illustrating an example of a data table linking a part of product ions to predicted mass values created in one embodiment of the present invention.
FIG. 13 is an explanatory diagram illustrating an example of a data table linking another part of product ions to predicted mass values created in one embodiment of the present invention.
FIG. 14 is an explanatory diagram illustrating an example of a data table linking nodes to scores created in one embodiment of the present invention.
FIG. 15 is an explanatory diagram illustrating an example of a data table linking edges to scores created in one embodiment of the present invention.
FIG. 16 is an explanatory diagram illustrating an example of a data table linking base deficient ions to predicted mass values created in one embodiment of the present invention.
FIG. 17 is an explanatory diagram illustrating an example of a data table linking edges to normalized scores created in one embodiment of the present invention.
FIG. 18 is an explanatory diagram illustrating an example of a data table linking nodes to updated scores created in one embodiment of the present invention.
FIG. 19 is an explanatory diagram illustrating an example of a directed graph created in one embodiment of the present invention.
FIG. 20 is an explanatory diagram illustrating an example of a mass spectrum including the results of matching processing created in one embodiment of the present invention.
FIG. 21 is an explanatory diagram illustrating another example of a directed graph created in one embodiment of the present invention.
FIG. 22 is an explanatory diagram illustrating yet another example of a directed graph created in one embodiment of the present invention.
FIG. 23 is an explanatory diagram illustrating a still further example of a directed graph created in one embodiment of the present invention.
FIG. 24 is an explanatory diagram illustrating yet another example of a directed graph created in one embodiment of the present invention.
FIG. 25 is an explanatory diagram illustrating another example of a directed graph created in one embodiment of the present invention.
FIG. 26 is an explanatory diagram illustrating an example of the results obtained by ranking, based on scores, candidate regions of an *Escherichia coli* genome in one embodiment of the present invention.
FIG. 27 is an explanatory diagram illustrating another example of the results obtained by ranking, based on scores, candidate regions of an *Escherichia coli* genome in one embodiment of the present invention.
FIG. 28 is an explanatory diagram illustrating an example of the results obtained by ranking, based on scores, candidate regions of a human genome in one embodiment of the present invention.
FIG. 29 is an explanatory diagram illustrating an example of a directed graph in the case of creating base composition sets by dividing a total base composition into three in one embodiment of the present invention.
FIG. 30 is an explanatory diagram illustrating another example of a directed graph in the case of creating base composition sets by dividing a total base composition into three in one embodiment of the present invention.

### BEST MODE FOR CARRYING OUT THE INVENTION

Hereinafter, an apparatus (hereinafter, referred to as "apparatus of the present invention"), a method (hereinafter, referred to as "method of the present invention"), and a program (hereinafter, referred to as "program of the present invention") for analysis of a base sequence and a base modification of a nucleic acid according to one embodiment of the present invention are described with reference to the figures. This embodiment describes an example where the method of the present invention is performed by using the apparatus of the present invention that operates in accordance with the program of the present invention.

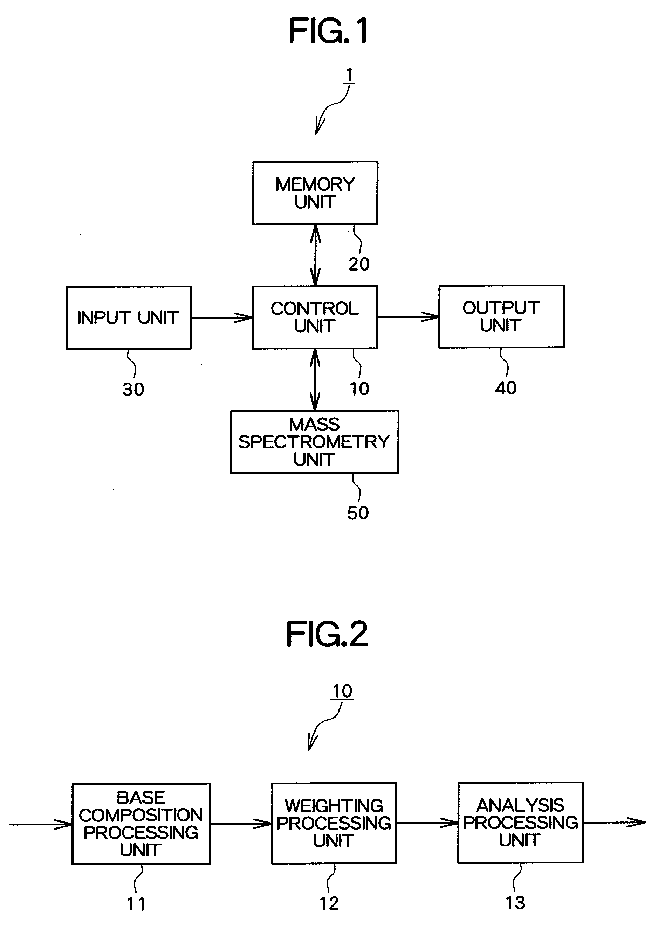

First, outlines of the apparatus of the present invention and the program of the present invention are described. The apparatus of the present invention may be constructed so as to include a computer in at least one part thereof. FIG. 1 is a block diagram illustrating a main structure of the apparatus 1 of the present invention. As shown in FIG. 1, the apparatus 1 of the present invention includes a control unit 10, a memory unit 20, an input unit 30, an output unit 40, and a mass spectrometry unit 50.

The control unit 10 may be embodied by an arithmetic device such as a CPU. The control unit 10 may operate in accordance with a program which has been stored in the memory unit 20, which allows the input or output of data between the memory unit 20, the input unit 30, the output unit 40, and the mass spectrometry unit 50. That is, the control unit 10 may store, in the memory unit 20, data relating to mass spectrometry received from the mass spectrometry unit 50, and may carry out predetermined arithmetic processing based on data read out from the memory unit 20 and data received from the input unit 30. Further, based on data relating to the results of arithmetic processing, the control unit 10 may display a predetermined image representing the results on the output unit 40. The control unit 10 may also output instruction data to the mass spectrometry unit 50 so that predetermined mass spectrometry can be carried out.

FIG. 2 is a function block diagram illustrating main processing to be performed by the control unit 10. As shown in FIG. 2, the control unit 10 functionally includes a base composition processing unit 11, a weighting processing unit 12, and an analysis processing unit 13. Specific contents of processing with the control unit 10 are described in detail later.

The memory unit 20 may be embodied by an external memory device such as a hard disk and a main memory device such as RAM and ROM. That is, for example, the memory unit 20 includes a hard disk, and stores, in the hard disk, a program (such as the program of the present invention, and a program with which the mass spectrometry unit 50 carries out mass spectrometry or analyzes mass spectrometry results) to be carried out by control unit 10, a database to be utilized in arithmetic processing by the control unit 10, and data relating to mass spectrometry received from the mass spectrometry unit 50 by the control unit 10. The memory unit 20 may also operate as a working memory for holding data that is utilized in processing to be performed by the control unit 10. That is, for example, the memory unit 20 includes RAM, and temporarily holds, in the RAM, data received from the input unit 30 and the mass spectrometry unit 50 by the control unit 10, and data produced in the course of arithmetic processing by the control unit 10.

The input unit 30 may be embodied by an input device such as a keyboard, a mouse, and a touchpad. The input unit 30 may receive input data from a user of the apparatus 1 of the present invention. The input unit 30 may also be embodied so as to include a drive device for a recording medium such as CD and DVD. In this case, the input unit 30 reads out data, which has been recorded on the recording media inserted into the drive device, and outputs the data to the control unit 10.

The output unit 40 may be embodied by a display device such as a liquid crystal display. Based on data received from the control unit 10, the output unit 40 may present a screen for displaying the results of processing performed by the control unit 10, and a screen for requiring a user to input data.

The mass spectrometry unit 50 may be embodied by a mass spectrometer such as QqTOF LC/MS/MS suitable for mass spectrometry of nucleic acids. The mass spectrometry unit 50 carries out mass spectrometry of nucleic acids of interest, and outputs data relating to the analysis results to the control unit 10.

The program of the present invention may make a computer carry out processing relating to the present invention. For example, the program of the present invention may make the apparatus of the present invention carry out processing relating to the method of the present invention. Further, the program of the present invention may be recorded on a computer-readable recording medium.

Next, the apparatus, the method, and the program of the present invention are described in detail. Here, there is described an example where an RNA fragment (hereinafter, referred to as "target fragment") obtained by cleaving an RNA molecule with a predetermined length using RNase A, which is one kind of ribonucleases, is used as a nucleic acid as an analytic target.

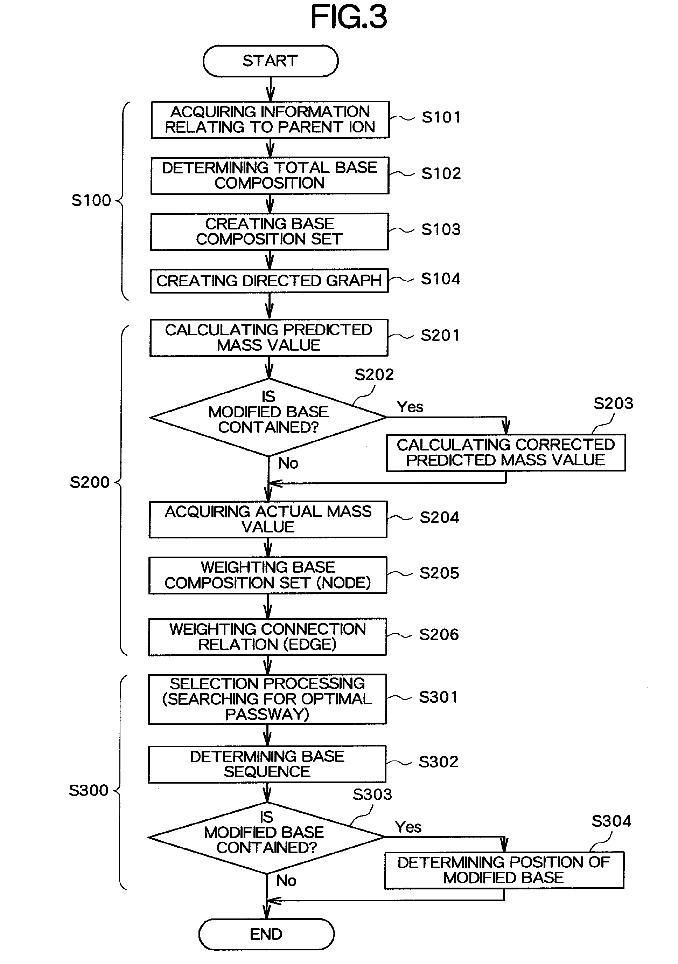

FIG. 3 is a flow diagram illustrating an example of main steps included in the method of the present invention. As shown in FIG. 3, the method of the present invention includes a first step S100, a second step S200, and a third step S300.

In the first step S100, the base composition processing unit 11 in the apparatus 1 of the present invention acquires information relating to a parent ion of a target fragment (S101). The parent ion may be produced from the target fragment by an ionization method suitable for mass spectrometry of nucleic acids such as an electrospray ionization (ESI) method and a matrix-assisted laser desorption/ionization (MALDI) method in a mass spectrometer included in the mass spectrometry unit 50.

As the information relating to the parent ion, for example, the mass-to-charge ratio (m/z value) and the valence of the parent ion, the kind of RNase used for preparing the target fragment, and the kind of modified base contained in the target fragment may be used. The m/z value and the valence of the parent ion may be measured with high accuracy by performing mass spectrometry of the parent ion by using an LC-QqTOF type mass spectrometer included in the mass spectrometry unit 50 within an error range of the mass spectrometer, for example.

The base composition processing unit 11 may receive such information relating to the parent ion from the mass spectrometry unit 50. It should be noted that the base composition processing unit 11 may also acquire the information relating to the parent ion by receiving the information via the input unit 30 from a user of the apparatus 1 of the present invention or by reading out the information from the memory unit 20.

Next, the base composition processing unit 11 determines the total base composition of the target fragment based on the information relating to the parent ion (S102). Here, the "base composition" consists of the kind of bases and the number of each of the bases contained in a nucleic acid molecule with a predetermined length, and is determined irrespective of the sequence order of each of the bases in the nucleic acidmolecule. The "total base composition" of the target fragment is a base composition as a whole of the target fragment.

In this embodiment, for example, when the target fragment consists of three adenine (A) residues, one uracil (U) residue, and two guanine (G) residues, and is free of cytosine (C), the total base composition of the target fragment is described as "A3G2U". In the description of the base composition, the order of bases to be described has no relation to the sequence order of bases in the target fragment.

In the step S102, the base composition processing unit 11 first calculates a molecular weight (mass) of the parent ion based on the information such as the m/z value and the valence of the parent ion. Then, the base composition processing unit 11 determines the total base composition of the target fragment based on the molecular weight of the parent ion.

That is, for example, when the memory unit 20 stores a database linking various base compositions of RNA fragments to their molecular weights, the base composition processing unit 11 matches the molecular weight of the parent ion with the molecular weight of each of the base compositions which have been stored in the database. Then, the base composition processing unit 11 extracts, from the base compositions which have been registered in the database, a base composition whose molecular weight coincides with or differs in a predetermined error range from the molecular weight of the parent ion, and determines the extracted base composition as the total base composition of the target fragment. It should be noted that the predetermined error range may be determined based on, for example, analysis accuracy depending on performance and analysis conditions of a mass spectrometer used for mass spectrometry of the parent ion.

Specifically, here, there is described an example where the base composition processing unit 11 calculates the molecular weight "2001.4 (Da)" based on the m/z value "999.7" and the valence "-2" of the parent ion, and determines the total base composition of the target fragment as "A3G2U" by database search based on the molecular weight.

Next, the base composition processing unit 11 creates a plurality of base composition sets based on the determined total base composition (S103). Each of the plurality of base composition sets consists of a set of partial base compositions.

Here, "a set of partial base compositions" are created as a combination of portions of the total base composition (partial base compositions) different from each other. That is, a plurality of partial base compositions consisting of a set of partial base compositions are a plurality of portions that are different from each other obtained by dividing the total base composition.

Further, each of the partial base compositions is specified by the kind of the bases, the number of each of the bases, and the positions of each of the partial base compositions in the target fragment. Thus, for example, when the positions of each of two partial base compositions in the target fragment differ from each other, the two partial base compositions differ from each other even if the two partial base compositions are identical to each other with respect to the kind of bases and the number of each of the bases consisting of each of the compositions. The positions of the partial base compositions may be determined as, for example, the terminal positions (5'-terminal position or 3'-terminal position) of the target fragment, or internal positions other than the termini.

Then, a set of partial base compositions at least contain a partial base composition at the 5'-terminal position (a partial base composition containing the 5'-terminal base) and a partial base composition at the 3'-terminal position (a partial base composition containing the 3'-terminus base). Specifically, for example, when a set of partial base compositions are created by dividing the total base composition "A3G2U" into two, the set of partial base compositions consists of the partial base composition "A" at the 5'-terminal position and the partial base composition "A2G2U" at the 3'-terminal position. Further, for example, when a set of partial base compositions are created by dividing the total base composition "A3G2U" into three, the set of partial base compositions consists of the partial base composition "A" at the 5'-terminal position, the partial base composition "U" at the 3'-terminal position, and the partial base composition "A2G2" at the internal position.

Such a set of partial base compositions may also be regarded as corresponding to a virtual set of product ions derived from a target fragment. That is, in the case of ionizing a target fragment to produce a parent ion, and further, dissociating the parent ion to produce product ions, each of the partial base compositions may be regarded as corresponding to a base composition of each of the product ions consisting of a set of product ions produced from one molecule of the parent ion. Thus, for example, a set of partial base compositions at least contain a partial base composition at the 5'-terminal position corresponding to a product ion containing a base at the 5'-terminus, and a partial base composition at the 3'-terminal position corresponding to a product ion containing a base at the 3-terminus. It should be appreciated that product ions corresponding to each of the partial base compositions may not be actually detected in such mass spectrometry, as be mentioned later.

Because each of the base composition sets consists of such a set of partial base compositions to be determined as mentioned above, the base composition as a whole thereof coincides with the total base composition. That is, the total of each of the partial base compositions constituting each of base composition sets coincides with the total base composition. Further, in this embodiment, the structure of each of the base composition sets is described so that a partial base composition on the 5'-terminal side and a partial base composition on the 3'-terminal side would be arranged via a colon (:) on the left side and on the right side, respectively. Specifically, for example, when base composition sets are created by dividing the total base composition "A3G2U" into two, the structure of a base composition set consisting of the partial base composition "A" at the 5'-terminal position and the partial base composition "A2G2U" at the 3'-terminal position is described as "A:A2G2U". Further, for example, when base composition sets are created by dividing the total base composition "A3G2U" into three, the structure of a base composition set consisting of the partial base composition "A" at the 5'-terminal position, the partial base composition "U" at the 3'-terminal position, and the partial base composition "A2G2" at the internal position is described as "A:A2G2:U".

Further, each of a plurality of base composition sets to be created from one total base composition consists of a set of partial base compositions that are different from each other. Here, for example, when at least one part of corresponding partial base compositions are different from each other in a comparison of a set of partial base compositions consisting of one of two base composition sets with a set of partial base compositions consisting of the other, the two base composition sets are formed of sets of partial base compositions that are different from each other.

It should be noted that the corresponding partial base compositions refer to partial base compositions that are identical with respect to the positions in the target fragment. For example, a partial base composition at the 5'-terminus contained in one of two base composition sets, and a partial base composition at the 5'-terminus contained in the other are corresponding partial base compositions. When two corresponding partial base compositions differ from each other in at least one of the kind of bases and the number of each of the bases, the two corresponding partial base compositions differ from each other.

In the step S103, the base composition processing unit 11 creates all or a part of possible combinations of partial base compositions to be expected from the total base composition based on data relating to the total base composition of the target fragment, a condition for dividing the total base composition, the kind of RNases used for preparing the target fragment, and the like, to thereby create a plurality of base composition sets that are different from each other. That is, for example, the base composition processing unit 11 calculates all of possible partial base composition sets in the case where a set of partial base compositions is created by dividing the total base composition into two, and generates data representing a plurality of base composition sets each consisting of a set of partial base compositions that are different from each other in the plurality of partial base composition sets. It should be noted that the base composition processing unit 11 may also acquire all or a part of data to be utilized for creating those partial base composition sets by receiving the data via the input unit 30 from a user or by reading out the data from the memory unit 20.

Here, because the target fragment is an RNA fragment prepared by cleavage treatment with RNase A as mentioned above, a base at the 3'-terminus of the target fragment may be expected to be uracil (U) or cytosine (C). Meanwhile, the total base composition of the target fragment determined in the above-mentioned step S102 is "A3G2U" and is free of cytosine (C). Therefore, the 3'-terminus of the target fragment may be determined as uracil (U). Further, here, an assumption is made that the parent ion is cleaved at one site (i.e., a set of product ions consisting of a product ion containing a base at the 5'-terminus and a product ion containing a base at the 3'-terminus are produced).

Thus, when the kind of bases at the terminus (5'-terminus or 3'-terminus) of the target fragment may be specified by the kind of ribonucleases used for preparing the target fragment as described above, the base composition processing unit 11 determines the kind of the base at the terminal position of the target fragment as "uracil (U) " based on the total base composition "A3G2U", the division number "2" of the total base composition, and the kind "RNase A" of the ribonucleases. Then, the base composition processing unit 11 creates a plurality of base composition sets based on the total base composition and the determination results of the terminal base. That is, the base composition processing unit 11 fixes the position of uracil (U) contained in the total base composition to the 3'-terminus, and then creates a plurality of partial base composition sets based on the remaining base composition in the total base composition.

Specifically, the base composition processing unit 11 calculates all of possible combinations of partial base compositions in the case where a partial base composition at the 3'-terminal position contains uracil (U), and creates eleven base composition sets being different from each other and having structures represented by "A:A2UG2", "G:A3UG", "A2:AUG2", "AG:A2UG", "G2:A3U", "A3:UG2", "A2G:AUG", "AG2:A2U", "A3G:UG", "A2G2:AU", and "A3G2:U", respectively.

Further, in the step S103, the base composition processing unit 11 creates a hierarchical structure having base composition sets hierarchized in ascending order of the number of bases in a partial base composition at at least one terminal position thereof. That is, based on, for example, instruction received via the input unit 30 fromauser, the base composition process ing unit 11 determines at least one of the 5'-terminus and the 3'-terminus as a focused terminus, and hierarchizes base composition sets based on the number of bases contained in a partial base composition at the position of the focused terminus. Specifically, here, the base composition processing unit 11 determines the 5'-terminus as the focused terminus, and classifies base composition sets having the same number of bases in a partial base composition at the 5'-terminal position into the same hierarchy. That is, the base composition processing unit 11 links predetermined numbers that are different from each other as the number of bases in a partial base composition at the 5'-terminal position to each hierarchy, and classifies all of possible base composition sets from the total base composition, the sets having a predetermined number of bases corresponding to each hierarchy in a partial base composition at the 5'-terminal position, into each hierarchy. Then, the base composition processing unit 11 ranks each of hierarchies in ascending order of the number of bases in partial base compositions at the 5'-terminal position constituting base composition sets belonging to each of the hierarchies.

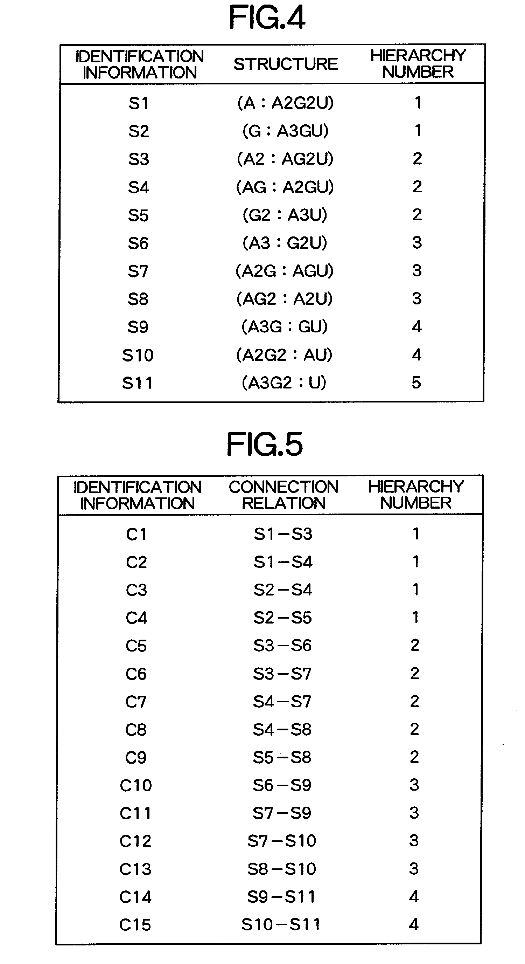

Then, as shown, for example, in FIG. 4, with respect to each of eleven base composition sets, the base composition processing unit 11 creates, as hierarchy data, a data table linking identification information S1 to S11 allocated to each of the base composition sets, the structure of each of the base composition sets, and hierarchy numbers to which each of the base composition sets belong, to each other.

As shown in FIG. 4, in the hierarchical structure, the number of bases contained in partial base compositions at the 5'-terminal position consisting base composition sets belonging to each hierarchy is increased one by one from one outermost hierarchy (a first hierarchy with a hierarchy number of "1") to the other outermost hierarchy (a fifth hierarchy with a hierarchy number of "5").

That is, two base composition sets S1 and S2, in each of which the number of bases in a partial base composition at the 5'-terminus is the smallest, that is the "1", are classified into a first hierarchy as one outermost hierarchy in a hierarchical structure, three base composition sets S3, S4, and S5, in each of which the number of bases is "2", that is larger by one, are classified into an internal second hierarchy, three base composition sets S6, S7, and S8, in each of which the number of bases is "3", that is larger again by one, are classified into a still deeper third hierarchy, two base composition sets S9 and S10, in each of which the number of bases is "4", that is larger again by one, are classified into a still deeper fourth hierarchy, and one base composition set S11 in which the number of bases is "5", that is the largest, is classified into a fifth hierarchy as the other outermost hierarchy in the hierarchical structure.

Meanwhile, in the hierarchical structure, when the number of bases in a partial base composition at the 3'-terminal position is focused, in contrast to the above, base composition sets are hierarchized in ascending order of the number of bases in a partial base composition at the 3'-terminal position from the fifth hierarchy to the first hierarchy. That is, in the hierarchical structure, base composition sets are hierarchized in ascending order of the number of bases in a partial base composition at the 5'-terminal position from one outermost hierarchy (first hierarchy) and in ascending order of a partial base composition at the 3'-terminal position from the other outermost hierarchy (fifth hierarchy).

In addition, in the step S103, the base composition processing unit 11 creates connection relations between each of base composition sets, and another base composition set having, at the terminal position, a partial base composition obtained by adding one base to a partial base composition at the terminal position of each of the base composition sets.

That is, the base composition processing unit 11 creates one connection relation between each of base composition sets belonging to one hierarchy to which base composition sets having a smaller number of bases in a partial base composition at the 5'-terminal position belong, out of adjacent hierarchies, and a base composition set belonging to the other hierarchy to which base composition sets having a larger number of bases in a partial base composition at the 5'-terminal position belong, and having a partial base composition obtained by adding one base to a partial base composition at the 5'-terminal position in base composition sets in the one hierarchy, as a partial base composition at the 5'-terminal position.

Specifically, among eleven hierarchized base composition sets as shown in FIG. 4, the base composition processing unit 11 creates a connection relation between, for example, a base composition set S1 belonging to the first hierarchy and having a partial base composition at the 5'-terminal position consisting of one adenine (A), and not only a base composition set S3 having a partial base composition at the 5'-terminal position created by adding another adenine (A) to one adenine (A) but also a base composition set S4 having a partial base composition at the 5'-terminal position created by adding one guanine (G) to one adenine (A), out of three base composition sets S3, S4, and S5 belonging to the adjacent second hierarchy.

Then, as shown in, for example, FIG. 5, with respect to each of the created connection relations, the base composition processing unit 11 creates, as connection data, a data table linking identification information C1 to C15 allocated to each connection relation, information for specifying a pair of base composition sets to be connected with each connection relation, and hierarchy numbers to which each connection relation belongs, to each other. In the example as shown in FIG. 5, the information for specifying a pair of base composition sets to be connected with each connection relation is a combination of identification information (see FIG. 4) which has been preliminarily allocated to each of base composition sets. Further, a hierarchy number to which each connection relation belongs is a smaller hierarchy number of hierarchy numbers to which each of base composition sets to be connected with each connection relation belongs (see FIG. 4).

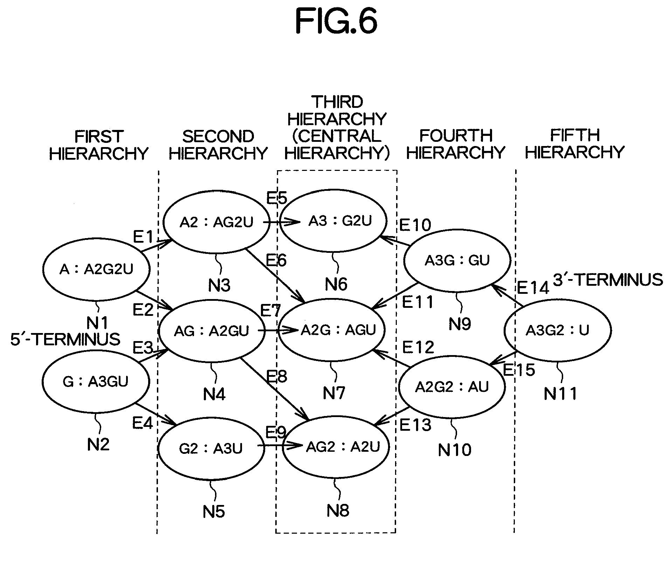

Next, the base composition processing unit 11 creates a directed graph having hierarchized nodes corresponding to each of base composition sets, and edges for connecting the nodes in a corresponding manner to the above-mentioned connection relations (S104). That is, the base composition processing unit 11 creates a directed graph visualizing connection relations between base composition sets based on such hierarchy data and connection data as mentioned above. Specifically, for example, the base composition processing unit 11 creates a directed graph as shown in FIG. 6.

In the directed graph as shown in FIG. 6, in accordance with the hierarchical structure as shown in FIG. 4, eleven nodes N1 to N11 corresponding to eleven base composition sets S1 to S11 as shown in FIG. 4 are hierarchically arranged in ascending order of the number of bases in a partial base composition at the 5'-terminal position consisting of a base composition set corresponding to each node from the outermost hierarchy (first hierarchy) at one terminus (left terminus in the figure) to the outermost hierarchy (fifth hierarchy) at the other terminus (right terminus in the figure), and in ascending order of the number of bases in a partial base composition at the 3'-terminal position consisting of a base composition set corresponding to each node from the fifth hierarchy to the first hierarchy. It should be noted that, hereinafter, in the directed graph, the terminus on the first hierarchy side is referred to as 5'-terminus, and the terminus on the fifth hierarchy side is referred to as 3'-terminus.

That is, in the directed graph, nodes N1 to N11 are hierarchically arranged so that the number of bases in a partial base composition at the 5'-terminal position would be increased one by one inward from the 5'-terminus, while the number of bases in a partial base composition at the 3'-terminal position would be increased one by one inward from the 3'-terminus.

Further, in the directed graph, a pair of nodes corresponding to a pair of base composition sets to be connected with a connection relation are connected with each of edges E1 to E15 corresponding to connection relations C1 to C15 as shown in FIG. 5. That is, in adjacent hierarchies, each of nodes corresponding to base composition sets belonging to one hierarchy to which base composition sets each having a smaller number of bases in a partial base composition at the 5'-terminal position belong, and a node corresponding to base composition sets belonging to the other hierarchy to which base composition sets each having a larger number of bases in a partial base composition at the 5'-terminal position belong, and having, as a partial base composition at the 5'-terminal position, a partial base composition obtained by adding one base to a partial base composition at the 5'-terminal position in the base composition sets in the one hierarchy are connected with one edge.

Further, in the directed graph, as shown in FIG. 6, each edge is directed from the 5'-terminal hierarchy and the 3'-terminal hierarchy to the third hierarchy (hierarchy surrounded by a dotted line in FIG. 6) which has been preliminarily determined as the central hierarchy. It should be noted that the base composition processing unit 11 may determine, as the central hierarchy, any hierarchy excluding the outermost hierarchies at both termini. That is, for example, the base composition processing unit 11 may determine, as the central hierarchy, a hierarchy in which the number of hierarchies from the outermost hierarchies at both termini is the same, or a hierarchy in which the number of hierarchies from one outermost hierarchy differs by only one from the number of hierarchies from the other outermost hierarchy. Further, for example, the base composition processing unit 11 may determine, as the central hierarchy, a hierarchy to be instructed by data received via the input unit 30 from a user.

Further, to the output unit 40, the base composition processing unit 11 may give an instruction to present the directed graph as an image to a user. In this case, the output unit 40 displays a directed graph as shown in FIG. 6 on a screen such as a liquid crystal display. In addition, in this case, the base composition processing unit 11 may receive correction instruction by a user from the input unit 30, to thereby correct the directed graph. For example, in the case of receiving an instruction to change the central hierarchy from a user, the base composition processing unit 11 creates a directed graph having the central hierarchy changed in accordance with the instruction.

Then, the base composition processing unit 11 outputs base composition data including information relating to a base composition set and a directed graph such as the above-mentioned hierarchy data and connection data to the weighting processing unit 12.

In the second step S200, for each of partial base compositions, a mass value which is predicted to be obtained in mass spectrometry of a corresponding product ion is first calculated (S201). Here, the mass value may be an m/z value or may also be a value obtained by subjecting the m/z value to deconvolution (i.e., value obtained by converting the m/z value to a 0 valence), for example. That is, the weighting processing unit 12 calculates a mass value (predicted mass value) of a product ion, which is the mass value being predicted to be obtained as analysis results when an assumption is made that the product ion whose base composition coincides with each partial base composition is subjected to mass spectrometry, based on base composition data received from the base composition processing unit 11.

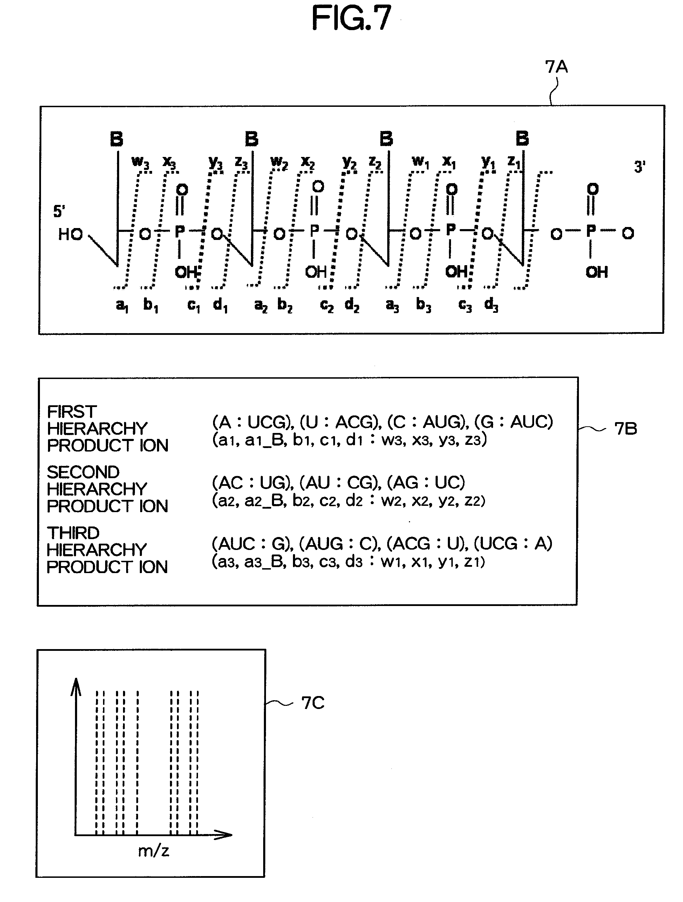

Here, in genenal, a parent ion (precursor ion) derived from a nucleic acid, as shown in FIG. 7A, is mainly cleaved at four sites of the respective phosphodiester bonds to produce various product ions. It should be noted that, here, a 4-mer RNA fragment ("B" reperesents a base) prepared with cleavage treatment using RNase A is shown as an example of the nucleic acid.

That is, as shown in FIGS. 7A and 7B, based on the nomenclature of McLuckey et al., the RNA fragment produces a, b, c, and d series of product ions as 5'-terminal product ions each containing a base at the 5'-terminus, and w, x, y, and z series of product ions as 3'-terminal product ions each containing a base at the 3'-terminus. Further, a series of product ions lack of a base (a_B) may be produced, for example.

Thus, when an RNA fragment is cleaved at a predetermined position between bases, each series of product ions may be considered to be produced for each of all possible base composition sets in each of which the number of bases in a partial base composition at the 5'-terminal position or the 3'-terminal position is a predetermined number to be determined based on a cleavage position. That is, when the RNA fragment has a total base composition of "AUCG" and is cleaved between the first base and the second base from the 5'-terminus, as shown in FIG. 7B, five kinds of 5'-terminal product ions "a1", "b1", "c1", "d1", and a1_B" and four kinds of 3'-terminal product ions "w3", "x3", "y3", and "z3" may be considered to be produced for each of all of possible base composition sets "A:UCG", "U:ACG", "C:AUG", and "G:AUC" in each of which the number of bases in a partial base composition at the 5'-terminal position is "1", for example. It should be noted that, as shown in FIG. 7A, an RNA fragment prepared with RNase A often has a hydroxy group (OH) at the 5'-terminus and has a phosphate group (PO₄H) at the 3'-terminus. The same is equally true of an RNA fragment prepared with RNase T. Thus, the kind of terminal functional group of a nucleic acid fragment may be estimated based on the kind of nuclease used for preparing the nucleic acid fragment, and the series of product ions to be produced may also be estimated based on information relating to ionization, such as easiness in ionization of the functional group.

So, in step S201, based on each of partial base compositions consisting of each base composition set and such various cleavage patterns as shown in FIG. 7A, the weighting processing unit 12 calculates a predicted mass value for at least one series of product ions corresponding to each partial base composition.

Specifically, the weighting processing unit 12 calculates, as a predicted mass value of a product ion corresponding to each partial base composition, an m/z value at which a peak corresponding to the product ion is theoretically expected to be detected. That is, the weighting processing unit 12 first calculates, for each partial base composition, a molecular composition of a product ion produced by cleavage at each of four cleavage sites as shown in FIG. 7A (the kind of atoms contained in the product ion and the number of each of the atoms). Next, the weighting processing unit 12 calculates a molecular weight of the product ion based on the molecular composition of the product ion. In addition, the weighting processing unit 12 calculates an m/z value of the product ion as a predicted mass value based on the molecular weight and the valence of the product ion. It should be noted that empirical findings may also be partially utilized in calculating the predicted mass value.

Then, the weighting processing unit 12 creates a theoretical spectrum as shown in FIG. 7C for each base composition set based on a predicted m/z value calculated for each of a plurality of partial base compositions consisting of each base composition set. That is, in each theoretical spectrum, one base composition set is linked to a predicted m/z value calculated for each series of product ions corresponding to each of partial base compositions consisting of the base composition set. Here, the weighting processing unit 12 calculates a predicted m/z value of a set of partial base compositions for each base composition set as shown in FIG. 4 or each node as shown in FIG. 6. That is, for example, with respect to a base composition set having a structure of "A2G:AGU", in the case of calculating all of base sequence candidates which may be possesed by the base composition set, arithmetic processing must be performed six times in view of all possible combinations of three base sequences "AAG", "AGA", and "GAA" which are possible as base sequences at the 5'-terminal position, and two base sequences "AGU" and "GAU" which are possible as base sequences at the 3'-terminal position. In contrast, the weighting processing unit 12 carries out arithmetic processing for one combination of partial base compositions with respect to the base composition set. Hence, in the present invention, a calculation amount may be effectively reduced compared with the case of calculating predicted m/z values for all base sequence candidates as mentioned above.

Next, the weighting processing unit 12 acquires a mass value actually obtained in mass spectrometry of product ions derived from a target fragment (actual mass value) (S204). It should be noted that, here, there is first described a case where no modified base is contained in a target fragment or a case where a base modification in a target fragment is not taken into consideration, and hence, descriptions about steps S202 and S203 as shown in FIG. 3 are omitted.

The actual mass value to be acquired by the weighting processing unit 12 may be, for example, a mass value obtained by ionizing a target fragment to obtain a parent ion, dissociating the obtained parent ion by a predetermined method inside of a mass spectrometer included in the mass spectrometry unit 50, to thereby produce product ions, and subjecting the product ions to mass spectrometry (MS/MS analysis) with the mass spectrometer. It should be noted that a dissociation method for the parent ion is not particularly limited as long as the method may produce product ions, and a Collision-Induced dissociation (CID) method may be preferably employed, for example. Further, any condition that produces product ions suitable for analysis may be appropriately set as dissociation conditions of the parent ion. For example, when CID is employed, collision energy suitable for efficiently generating a preferred series of product ions may be determined as one of the dissociation conditions. The weighting processing unit 12 may receive, for example, mass spectrum data actually obtained for product ions from the mass spectrometry unit 50 (actual spectrum), to thereby acquire an actual m/z value contained in the actual spectrum data as an actual mass value. Further, the weighting processing unit 12 may also acquire the actual m/z value by receiving the value via the input unit 30 from a user or by reading out the value from the memory unit 20.

Next, the weighting processing unit 12 imparts a weight to each of base composition sets based on a comparison between an predicted mass value and an actual mass value (S205). That is, based on, for example, a peak intensity corresponding to an actual m/z value having a difference from a predicted m/z value, the difference satisfying a predetermined condition, the weighting processing unit 12 imparts a weight to a node of a base composition set containing a partial base composition corresponding to the predicted m/z value.

Specifically, the weighting processing unit 12 first judges whether or not there exists, in actual m/z values corresponding to peaks contained in an actual spectrum, an actual m/z value having a difference from each predicted m/z value contained in a theoretical spectrum, the difference being equal to or less than a predetermined threshold. It should be noted that the threshold may be determined based on an allowable error of a mass spectrometer used for obtaining the actual m/z value, for example.

Then, when a difference between a certain predicted m/z value and a certain actual m/z value is equal to or less than a threshold, the weighting processing unit 12 judges that the prediced m/z value and the actual m/z value coincide with each other, that is, a peak corresponding to the predictedm/z value is hit in the actual spectrum. In this case, the weighting processing unit 12 determines, in the actual spectrum, a partial weight of a partial base composition corresponding to the predicted m/z value, based on the intensity of the detected peak (hit peak) at the position of the actual m/z value coincident with the predicted m/z value. Then, the weighting processing unit 12 imparts, as a weight of each node, a total of a partial weight determined for each of a plurality of partial base compositions corresponding to each node.

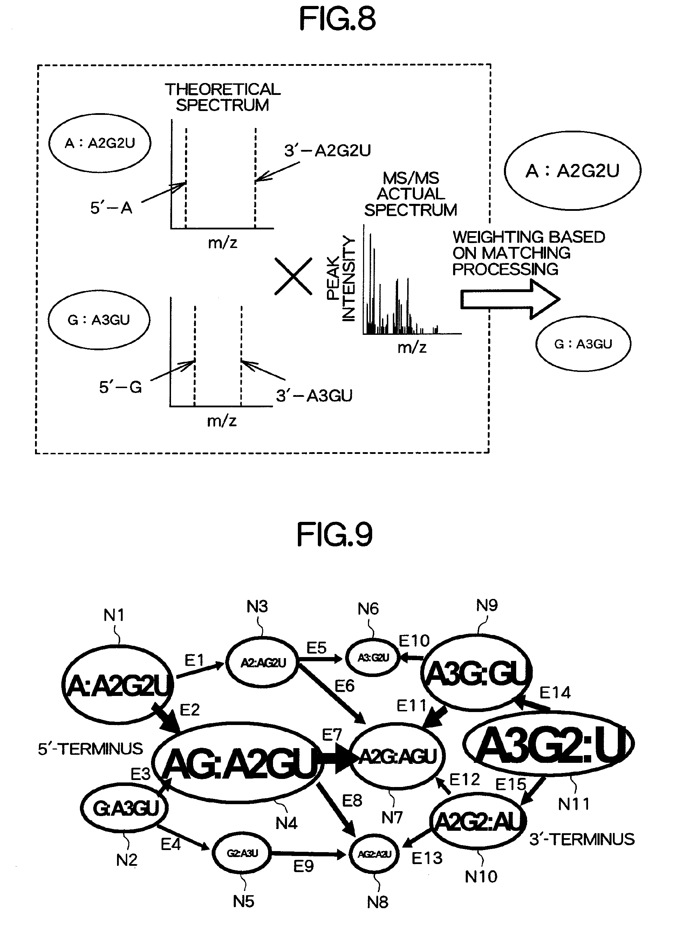

FIG. 8 is a conceptual diagram illustrating an example of weighting processing for each node based on the comparison between a predicted mass value and an actual mass value. As shown in FIG. 8, the weighting processing unit 12 creates a theoretical spectrum for each of a base composition set S1 having a structure of "A:A2G2U" and a base composition set S2 having a structure of "G:A3GU". Each theoretical spectrum specifies a predicted m/z value calculated for each partial base composition consisting of each base composition set. Then, the weighting processing 12 performs matching processing for matching each theoretical spectrum with an actual spectrum observed for product ions.

That is, the weighting processing unit 12 first judges whether or not any one of actual m/z values as peaks contained in an actual spectrum coincides with any one of a first predicted m/z value calculated for the partial base composition "A" at the 5'-terminal position and a second predicted m/z value calculated for the partial base composition "A2G2U" at the 3'-terminal position contained in a theoretical spectrum of the base composition set S1.

Then, the weighting processing unit 12 performs weighting for each node based on the results of the matching processing. That is, in the case of, for example, detecting both a first actual m/z value coincident with a first predict m/z value and a second actual m/z value coincident with a second predicted m/z value, the weighting processing unit 12 determines, as a weight of a node corresponding to the base composition set S1, a total of the intensity of a peak detected at the first actual m/z value and the intensity of a peak detected at the second actual m/z value.

Meanwhile, in the case of, in similar matching processing, judging that one of actual m/z values as peaks contained in an actual spectrum coincides with a first predicted m/z value calculated for the partial base composition "G" at the 5'-terminal position, but no actual m/z value coincides with a second predicted m/z value calculated for the partial base composition "A2G2U" at the 3'-terminal position, both the predicted m/z values being contained in a theoretical spectrumof the base composition set S2, the weighting processing unit 12 determines, as a weight of a node corresponding to the base composition set S2, only the intensity of a peak detected at the actual m/z value coincident with the first predicted m/z value. The weighting processing unit 12 carries out such weighting processing for all nodes.

Then, the weighting processing unit 12 produces, as node weight data, a data table linking identification information N1 to N11 (see FIG. 6) allocated to each node, a base composition set corresponding to each node, and a weight imparted to each node to each other, for example.

In addition, the weighting processing unit 12 updates a directed graph so that each node will be displayed in a mode depending on a weight of each node. That is, as shown in, for example, FIG. 9, the weighting processing unit 12 creates such a directed graph that each node is displayed in a size depending on its weight. In the directed graph as shown in FIG. 9, the node is larger in size as the weight is larger. The weighting processing unit 12 produces directed graph data for drawing such a directed graph as shown in FIG. 9 and outputs the directed graph data to the output unit 40, and the output unit 40 displays the directed graph on a screen such as a liquid crystal display. A user to whom such a directed graph has been presented may visually understand the results of weighting processing for each node.

Next, the weighting processing unit 12 imparts a weight to each of connection relations based on a weight imparted to a pair of base composition sets connected with each of the connection relations (S206). That is, for example, in the directed graph as shown in FIG. 9, the weighting processing unit 12 imparts a weight to each edge based on a total of a weight imparted to each of two nodes to be connected to both termini of each edge.

Specifically, for example, based on the above-mentioned node weight data, the weighting processing unit 12 imparts, as a weight of edge E2, a total of a weight imparted to a node N1 connected to one terminus of the edge E2 as shown in FIG. 9, and a weight imparted to a node N4 connected to the other. The weighting processing unit 12 carries out such weighting processing for all edges.

Then, the weighting processing unit 12 produces, as edge weight data, a data table linking identification information E1 to E15 allocated to each edge, information for specifying a pair of nodes connected with each edge, and a weight imparted to each edge, to each other, for example.

In addition, the weighting processing unit 12 updates a directed graph so that each edge will be displayed in a mode depending on a weight of each edge. That is, as shown in, for example, FIG. 9, the weighting processing unit 12 creates such a directed graph that each edge is displayed in a thickness depending on its weight. In the directed graph as shown in FIG. 9, the edge is larger in thickness as the weight is larger.

It should be noted that the weighting processing unit 12 may also normalize a weight of the edge. In this case, the weighting processing unit 12 normalizes a weight of each edge by dividing a weight of each edge belonging to each hierarchy by the maximum weight imparted to edges belonging to each hierarchy, for example. As a result, a weight represented by a value ranging from 0 to 1 may be imparted to each edge, for example. The weighting processing unit 12 outputs data such as node weight data and edge weight data representing the results of weighting processing as mentioned above to the analysis processing unit 13.

In the third step S300, the analysis processing unit 13 performs selection processing for selecting one of base composition sets belonging to each hierarchy while following connection relations from both of the outermost hierarchies in a hierarchical structure based on a weight imparted to each of base composition sets (S301). That is, for example, in the directed graph as shown in FIG. 9, the analysis processing unit 13 sequentially selects one of nodes belonging to each hierarchy while following edges inward from both termini of the 5'-terminus or the 3'-terminus in the directed graph based on a weight imparted to each node. It should be noted that, here, there is described an example where the analysis processing unit 13 performs selection processing by applying dynamic programming and searching for an optimum route in a directed graph.

Further, in general, there is a tendency for, in mass spectrometry of product ions produced by dissociation of a parent ion, a product ion of a short fragment containing a base at the 5'-terminus or a base at the 3'-terminus to be detected with high intensity. Thus, in mass spectrometry, the actual results of a short product ion containing a terminal base often have higher reliability compared with the actual results of a longer product ion.

So, in a directed graph, the analysis processing unit 13 follows edges while updating a weight of each node based on dynamic programming from the first hierarchy at the 5'-terminus to which a base composition set having the smallest number of bases in a partial base composition at the 5'-terminal position belongs, and the fifth hierarchy at the 3'-terminus to which a base composition set having the smallest number of bases in a partial base composition at the 3'-terminal position belongs as both the outermost hierarchies to the third hierarchy preliminarily determined as the central hierarchy, to thereby determine, as an optimum route, a route having the maximum weight from the 5'-terminus or the 3'-terminus to each node in the directed graph.

Specifically, the analysis processing unit 13 first determines, as an optimum route to each node, a route to which the maximum weight has been imparted out of routes from a node at the 5'-terminus or the 3'-terminus in a directed graph to each node, in accordance with a direction of edges. Then, the analysis processing unit 13 performs weight update processing for changing a weight of each node imparted by the weighting processing unit 12 as mentioned above (initial weight) to a weight of an optimum route to each node (updated weight). It should be noted that a weight of a route from a terminal node to each node in the directed graph may be a total of weights of each of edges which have been passed in an area from the terminal node to each node, for example.

The analysis processing unit 13 sequentially carries out the weight update processing for each node belonging to each hierarchy from the first hierarchy at the 5'-terminus and the fifth hierarchy at the 3'-terminus to the central third hierarchy. The search processing for an optimum route may be represented by using, for example, the Belman's formula well known in dynamic programming. That is, here, k is defined as a hierarchy number of the central hierarchy, and the function g (x) is defined as a function for returning a hierarchy number of a node x. Further, in a region from the outermost hierarchy at the 5'-terminus to the central hierarchy, nodes m and n which have been connected to each other with an edge satisfy the condition of g(n)<g(m)≤k. Then, when f (m) is defined as the maximum score from a node at the 5'-terminus to the node m, and Wnm is defined as a weight of an edge with which the nodes n and m have been connected to each other, the equation f(m)=max[Wnm+f(n)] is satisfied. Similarly, in a region from the outermost hierarchy at the 3'-terminus to the central hierarchy, nodes m and n which have been connected to each other with an edge satisfy the condition of g(n)>g(m)≥k. When f (m) is defined as the maximum score from a node at the 3'-terminus to the node m, and Wnm is defined as a weight of an edge with which the nodes n and m have been connected to each other, the equation f(m)=max[Wnm+f(n)] is satisfied. It should be noted that the analysis processing unit 13 applies forward dynamic programming for a node in the outermost hierarchy at the 5'-terminus in a directed graph to a node in the central hierarchy, and applies backward dynamic programming for a node in the outermost hierarchy at the 3'-terminus to a node in the central hierarchy.

Specifically, the analysis processing unit 13 determines weights of two nodes N1 and N2 belonging to the first hierarchy at the 5'-terminus in the directed graph as shown in FIG. 9 as 0. Next, in updating a weight of, for example, a node N4 out of nodes N3, N4, and N5 belonging to the second hierarchy, the analysis processing unit 13 compares a first candidate weight, as a total of a weight of an edge E2 which has been connected to the node N4 on the 3'-terminal side and a weight of a node N1 which has been connected to the 5'-terminal side of the edge E2, with a second candidate weight, as a total of a weight of another edge E3 which has been connected to the node N4 on the 3'-terminal side and a weight of a node N2 which has been connected to the 5'-terminal side of the edge E3. Then, the analysis processing unit 13 determines the first candidate weight as an updated weight of the node N4 when judging that the first candidate weight is larger in those candidate weights. Then, the analysis processing unit 13 holds the determined updated weight and information for specifying the edge E2 to which the updated weight has been imparted (e.g., as shown in FIG. 5, identification information "C2" imparted to a connection relation C2 corresponding to the edge E2, and identification information "E2" imparted to the edge E2) each linked to the node N4.

The analysis processing unit 13 also performs such weight update processing for other nodes N3 and N5 in the second hierarchy. Further, similarly, the analysis processing unit 13 sequentially carries out weight update processing for a node N11 belonging to the fifth hierarchy at the 3'-terminus, and then weight update processing for two nodes N9 and N10 belonging to the fourth hierarchy.

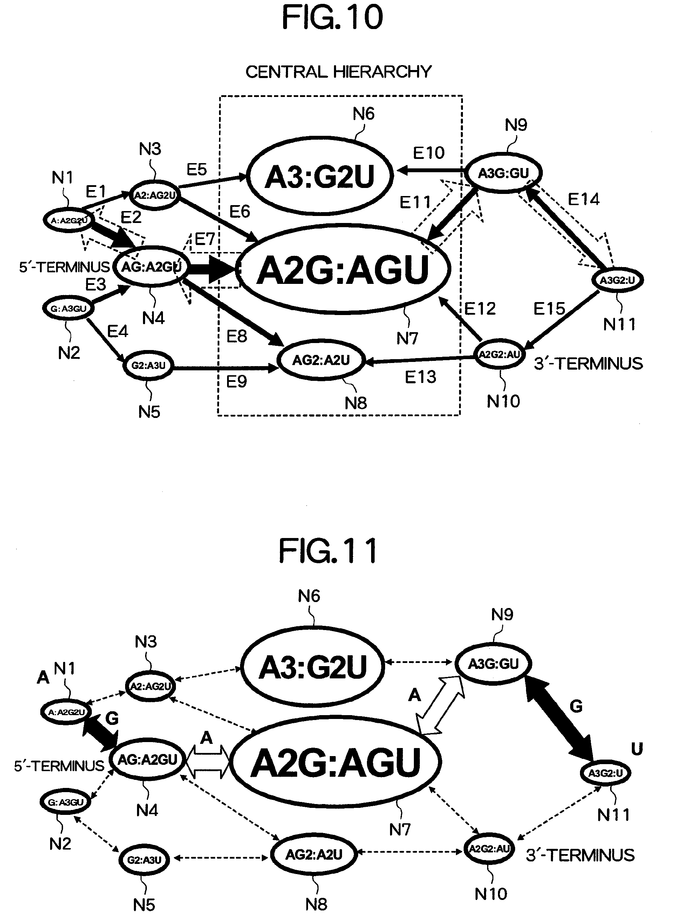

Then, the analysis processing unit 13 performs weight update processing for nodes N6, N7, and N8 belonging to the third hierarchy designated as the central hierarchy, based on a weight of an optimum route from the 5'-terminal side in a directed graph to each of the nodes N6, N7, and N8, and a weight of an optimum route from the 3'-terminal side in a directed graph to each of the nodes N6, N7, and N8. That is, for example, the analysis processing unit 13 changes a weight of a node N7 belonging to the central hierarchy to an updated weight obtained by totalizing a weight of an optimum route from the 5'-terminal side to the node N7, the optimum route including a node N1, an edge E2, a node N4, and an edge E7 connected to each other, and a weight of an optimum route from the 3'-terminal side to the node N7, the optimum route including a node N11, an edge E14, a node N9, and an edge E11 connected to each other. Specifically, the analysis processing unit 13 calculates an updated weight of the node N7 by, for example, totalizing a partial updated weight on the 5'-terminal side obtained by totalizing an updated weight of the node N4 and a weight of the edge E7, and a partial updated weight on the 3'-terminal side obtained by totalizing an updated weight of the node N9 and a weight of the edge E11. Then, the analysis processing unit 13 holds an updated weight obtained by totalizing the partial updated weights, information for specifying the edge E7 to which a partial updated weight on the 5'-terminal side has been imparted, and information for specifying the edge E11 to which a partial updated weight on the 3'-terminal side has been imparted, each linked to the node N7. Similarly, the analysis processing unit 13 also carries out weight update processing for other nodes N6 and N8 belonging to the central hierarchy. As described above, the analysis processing unit 13 performs weight update processing for each node depending on weights imparted to a node and an edge on a more terminal side from both termini to the center in a directed graph. Further, the analysis processing unit 13 may update a directed graph so that each node will be displayed in a mode depending on an updated weight of each node. That is, in this case, as shown in, for example, FIG. 10, the analysis processing unit 13 creates such a directed graph that each node is displayed in a size depending on its updated weight. In the directed graph as shown in FIG. 10, the node is larger in size as the updated weight is larger. The analysis processing unit 13 produces directed graph data for drawing such a directed graph as shown in FIG. 10 and outputs the directed graph data to the output unit 40, and the output unit 40 displays the directed graph on a screen such as a liquid crystal display. A user to whom such a directed graph has been presented may visually understand the results of weighting update processing for each node. It should be noted that the analysis processing unit 13 may store an updated weight and a weight before update in the memory unit 20, respectively.

Then, next, as shown with a dashed arrow in FIG. 10, the analysis processing unit 13 performs trace back processing for following an optimum route from the central hierarchy (third hierarchy surrounded by a dashed line) to the outermost hierarchies at both termini in a directed graph, to thereby select one of nodes belonging to each hierarchy.

That is, the analysis processing unit 13 first compares updated weights of three nodes N6, N7, and N8 belonging to the central hierarchy with each other, and selects the node N7 to which the largest updated weight has been imparted. Next, the analysis processing unit 13 selects, from edges E6 and E7 belonging to the second hierarchy at the 5'-terminal side, the 3'-terminal side of each of which has been connected to the node N7 selected in the central hierarchy, the edge E7 which has been preliminarily linked to the node N7 as mentioned above and to which a partial updated weight on the 5'-terminal side has been imparted, and selects, from nodes N3, N4, and N5 belonging to the second hierarchy, the node N4 which has been connected to the 5'-terminal side of the selected edge E7. Further, similarly, the analysis processing unit 13 selects, from edges E11 and E12 belonging to the fourth hierarchy on the 3'-terminal side, the edge E11 which has been preliminarily linked to the node N7 as mentioned above and to which a partial updated weight on the 3'-terminal side has been imparted, and selects, from nodes N9 and N10 belonging to the fourth hierarchy, the node N9 which has been connected to the 3'-terminal side of the selected edge E11. In addition, similarly, the analysis processing unit 13 selects, from edges E2 and E3 belonging to the first hierarchy at the 5'-terminus, the edge E2 which has been preliminarily linked to the node N4 as mentioned above and to which an updated weight has been imparted, and selects, from nodes N1 and N2 belonging to the first hierarchy, the node N1 which has been connected to the 5'-terminal side of the selected edge E2. Meanwhile, the analysis processing unit 13 selects the node N11 because only the node N11 belongs to the fifth hierarchy at the 3'-terminus.

As described above, the analysis processing unit 13 performs trace back processing based on dynamic programming from the central hierarchy to the outermost hierarchies at both termini in a directed graph, and definitely determines an optimum route which has been passed from the outermost hierarchies at both termini to the node N7 selected in the central hierarchy. That is, the analysis processing unit 13 selects a node belonging to each hierarchy on an optimum route one by one by the trace back processing. As a result, in the directed graph as shown in FIG. 10, the analysis processing unit 13 selects the node N1 in the first hierarchy at the 5'-terminus, the node N4 in the second hierarchy, a node N7 in the central hierarchy, the node N9 in the fourth hierarchy, and the node N11 in the fifth hierarchy at the 3'-terminus, respectively.

Next, the analysis processing unit 13 sequentially determines a base sequence from both termini of the target fragment based on the results of selection processing as mentioned above (S302). That is, the analysis processing unit 13 carries out base specifying processing involving comparing corresponding partial base compositions between two base composition sets selected in each of adjacent hierarchies, and specifying a base forming a difference in the corresponding partial base compositions, from the first hierarchy to the third hierarchy for a partial base composition at the 5'-terminal position, and from the fifth hierarchy to the third hierarchy for a partial base composition at the 3'-terminal position, respectively, to thereby determine a base sequence of the target fragment sequentially from both sides of the 5'-terminal side and the 3'-terminal side.

Specifically, as shown in FIG. 11, the analysis processing unit 13 specifies a base forming a difference between corresponding partial base compositions between nodes to be adjacently arranged on the optimum route in a directed graph, the nodes having been definitely determined by the above-mentioned selection processing, sequentially from the 5'-terminal side and the 3'-terminal side as both terminal sides in the directed graph.

That is, the analysis processing unit 13 first determines a base at the 5'-terminus of the target fragment as adenine (A) based on the fact that a partial base composition at the 5'-terminal position of the node N1 selected in the first hierarchy at the 5'-terminus is one adenine (A). Next, the analysis processing unit 13 compares a partial base composition at the 5'-terminal position of the node N1 selected in the first hierarchy with a partial base composition at the 5'-terminal position of the node N4 selected in the second hierarchy adjacent to the internal side (3'-terminal side) of the first hierarchy, to thereby specify guanine (G) as a base forming a difference between those partial base compositions. That is, the analysis processing unit 13 determines the second base from the 5'-terminus of the target fragment (i.e., base adjacent to the 3'-terminal side of adenine (A)) as guanine (G).

In addition, similarly, the analysis processing unit 13 determines a base at the 3'-terminus of the target fragment as uracil (U) based on the fact that a partial base composition at the 3'-terminal position of the node N11 selected in the fifth hierarchy at the 3'-terminus is one uracil (U). Next, the analysis processing unit 13 compares a partial base composition at the 3'-terminal position of the node N11 selected in the fifth hierarchy with a partial base composition at the 3'-terminal position of the node N9 selected in the fourth hierarchy adjacent to the internal side (5'-terminal side) of the fifth hierarchy, to thereby specify guanine (G) as a base forming a difference between those partial base compositions. That is, the analysis processing unit 13 determines the second base from the 3'-terminus of the target fragment (i.e., base adjacent to the 5'-terminal side of uracil (U)) as guanine (G).

In addition, similarly, the analysis processing unit 13 specifies adenine (A) as a base forming a difference between a partial base composition at the 5-terminal position of the node N4 selected in the second hierarchy and a partial base composition at the 5'-terminal position of the node N7 selected in the central third hierarchy, while specifying adenine (A) as a base forming a difference between a partial base composition at the 3'-terminal position of the node N9 selected in the fourth hierarchy and a partial base composition at the 3'-terminal position of the node N7 selected in the central third hierarchy. As a result, the analysis processing unit 13 determines the total base sequence of the target fragment as "AGAAGU". Then, to the output unit 40, the analysis processing unit 13 gives an instruction to present an interface screen including an image representing the determination results of the base sequence to a user. That is, as shown, for example, in FIG. 11, the analysis processing unit 13 may produce directed graph data for drawing such a directed graph that a node selected in each hierarchy is displayed in a distinguishable manner from the other nodes, and an edge for connecting nodes selected in each hierarchy is displayed in a distinguishable manner from the other edges, and output the directed graph data to the output unit 40. As shown in FIG. 11, the directed graph data may include data for displaying a base forming a difference in partial base composition between nodes selected in each hierarchy in a corresponding manner to the edge for connecting nodes. Further, for example, the analysis processing unit 13 may present, to a user, a directed graph displaying a weight before update of each node and the determination results of the base sequence linked to each other. That is, in this case, for example, the analysis processing unit 13 produces directed graph data for drawing such a directed graph that each node is displayed in a size depending on its weight before update, a node selected in each hierarchy is displayed in a distinguishable manner from other nodes, an edge for connecting nodes selected in each hierarchy is displayed in a distinguishable manner from the other edges, and a base forming a difference in partial base composition between nodes selected in each hierarchy is displayed in a corresponding manner to the edge for connecting nodes, and outputs the directed graph data to the output unit 40, and the output unit 40 displays the directed graph on a screen such as a liquid crystal display. A user to whom such a directed graph has been presented may visually understand the analysis results of the base sequence by linking the results to a weight before update and a weight after update of each node, a weight of an edge, and the like. Thus, for example, the user may easily determine an instruction to correct an analysis condition, and the apparatus 1 of the present invention, which has received an input of correction instruction, may present the results of further analysis performed in accordance with the correction instruction to the user.

As mentioned above, according to the present invention, a nucleic acid may be analyzed quickly and precisely. That is, in, for example, such technology as producing all possible base sequence candidates which are expected from the total base composition, the calculation amount is drastically increased as the number of bases is increased. In contrast, in the present invention, base composition sets each consisting of a set of partial base compositions are produced without producing all of such possible base sequence candidates, and hence the increase in the calculation amount due to the increase in the number of bases may be effectively inhibited. Further, according to the present invention, it is not necessary to search a database including registered known nucleic acid base sequences, and the total base sequence of a nucleic acid may be in silico determined quickly by arithmetic processing based on mass spectrometry results (de novo sequencing).

Further, in the present invention, selection processing for base composition sets is performed inward from the outermost hierarchy in a hierarchical structure, based on weights of base composition sets. Therefore, a base sequence of a nucleic acid may be determined from the terminus by effectively utilizing mass spectrometry results of product ions derived from the terminus with high reliability, with the result that an analysis with high reliability may be performed.

The present invention provides a basic technology in a life science such as development of a novel diagnostic technology using RNA as a target, and development of an analysis technology for an RNA-protein network, and thus may be expected to have extremely wide applications.

Further, according to the present invention, a base modification contained in a target fragment may also be analyzed. Processing in this case is described with reference to FIG. 3. For content overlapping with the above-mentioned content, a detailed description thereof is omitted.

First, in the first step S100, thecontentofabasemodification contained in a target fragment is estimated. That is, for example, in the above-mentioned step S102, the base composition processing unit 11 estimates that the target fragment contains a predetermined modification portion when the presence of the predetermined modification portion (e.g., a functional group such as a methyl group) must be considered in determining the total base composition of the target fragment based on the observed molecular weight of the target fragment. It should be noted that, for example, the fact that the target fragment contains a predetermined modification portion may also be estimated from the results of other analysis techniques such as elution time of the target fragment in liquid chromatography.

The base composition processing unit 11 produces modification data representing the content of a modification portion (i.e., the kind, molecular weight, number, valence, and the like of a modification group) when judging that the target fragment contains the modification portion, and outputs the modification data to the weighting processing unit 12.

Next, in the second step S200, mass spectrometry results of each product ion, that are predicted when an assumption is made that the target fragment contains a modified base, are created. That is, the weighting processing unit 12 calculates, in the above-mentioned step S201, a first predicted mass value to be predicted when an assumption is made that a product ion corresponding to each partial base composition is free of any modified base, while judges, in the step S202, whether or not the target fragment contains a modified base. Here, the weighting processing unit 12 judges that the target fragment contains a modified base when receiving modification data from the base composition processing unit 11 (Yes in S202), and calculates, in step S203, a second predicted mass value (corrected predicted mass value) to be predicted when an assumption is made that the modified base is contained in a product ion corresponding to each partial base composition.

Specifically, for example, the weighting processing unit 12 corrects a first predicted mass value based on the kind, molecular weight, number, valence, and the like of a modification group contained in modification data, to thereby calculate a second predicted mass value. That is, for example, in the case of calculating, in the step S201, a first predicted m/z value based on a molecular weight of a product ion corresponding to each partial base composition, the weighting processing unit 12 calculates, in step S203, a second predicted m/z value to be predicted when an assumption is made that the product ion contains a modification group, based on a corrected molecular weight obtained by adding a molecular weight of the modification group to the molecular weight of the product ion.

Then, in the step S205, the weighting processing unit 12 performs weighting for each node, based on a comparison of each of those first predicted m/z value and second predicted m/z value with an actual m/z value. That is, the weighting processing unit 12 searches whether or not there exists, in an actual spectrum, an actual m/z value close to the first predicted m/z value or the second predicted m/z value.

Then, the weighting processing unit 12 calculates, based on the first predicted m/z value and a predetermined error range, a first m/z range from a lower limit value, which is smaller than the first predicted m/z value by a predetermined value, to an upper limit value which is larger than the first predicted m/z value by a predetermined value, and judges whether or not an actual m/z value within the first m/z range is contained in the actual spectrum. Here, the weighting processing unit 12 determines, in the case of detecting an actual m/z value (first actual m/z value) within the first m/z range, a first tentative weight based on, for example, the intensity of a peak detected at the first actual m/z value out of peaks contained in the actual spectrum. Meanwhile, similarly, the weighting processing unit 12 calculates, based on the second predicted m/z value and a predetermined error range, a second m/z range from a lower limit value which is smaller than the second predicted m/z value by a predetermined value to an upper limit value which is larger than the second predicted m/z value by a predetermined value, and judges whether or not an actual m/z value within the second m/z range is contained in the actual spectrum. Then, the weighting processing unit 12 determines, in the case of detecting an actual m/z value (second actual m/z value) within the second m/z range, a second tentative weight based on, for example, the intensity of a peak detected at the second actual m/z value out of peaks contained in the actual spectrum. Next, the weighting processing unit 12 performs weighting for a corresponding node based on a larger tentative weight in a comparison of the first tentative weight and the second tentative weight. That is, for example, the weighting processing unit 12 judges that a product ion of interest contains a modified base, in the case of judging that the second tentative weight is larger than the first tentative weight. Then, in this case, the weighting processing unit 12 performs weighting for a corresponding node based on, for example, the intensity of a peak detected at the second actual m/z value out of peaks contained in the actual spectrum. It should be noted that the weighting processing unit 12 does not perform weighting for the first m/z value or the second m/z value, in the case of judging that no observed m/z value within the first m/z range or within the second m/z range is contained in the actual spectrum. Further, the above-mentioned predetermined error range may be determined based on, for example, analysis accuracy depending on performance, analysis conditions, and the like of a mass spectrometer used for mass spectrometry of the parent ion.

Further, in the case of judging that the product ion contains a modified base, the weighting processing unit 12 links modification tag data indicating that the modified base is contained to a partial base composition corresponding to the product ion, and holds the partial base composition.

Then, in the third step S300, the analysis processing unit 13 judges whether or not the target fragment contains a modified base (S303) after the determination of the base sequence of the target fragment (S302). Then, in the case of judging that the target fragment contains a modified base (Yes in S303), the analysis processing unit 13 determines a modified base position based on the position of a partial base composition to which modification tag data has been linked out of partial base compositions consisting of each base composition set (S304).

That is, for example, the analysis processing unit 13 specifies, as a modified base, a base forming a difference in the partial base composition between base composition sets selected in adjacent hierarchies when the position of a partial base composition to which modification tag data has been linked moves, in the base composition sets selected by the above-mentioned selection processing, between the adjacent hierarchies in a hierarchical structure.

That is, for example, in the directed graph as shown in FIG. 11, when modification tag data has been linked to the partial base composition "A2GU" at the 3'-terminal position in the base composition set "AG:A2GU" corresponding to the node N4 selected in the second hierarchy, while modification tag data has been linked to the partial base composition "A2G" at the 5' -terminal position in the base composition set "A2G:AGU" corresponding to the node N7 selected in the third hierarchy, the analysis processing unit 13 judges that the position of a partial base composition to which modification tag data has been linked has moved from the 3'-terminal position to the 5'-terminal position.

Then, in this case, the analysis processing unit 13 compares the partial base composition "A2G" at the 5'-terminal position according to the node N7 to which modification tag data has been newly linked in the internal hierarchy (third hierarchy) along with the increase in the number of bases, with the partial base composition "AG" at the 5'-terminal position according to the node N4 in the hierarchy on the terminal side, and specifies, as a modified base, the third adenine (A) from the 5'-terminus forming a difference between those partial base compositions. Further, in the same manner as the case of specifying a modified base as described above, for example, a terminal structure of the target fragment maybe specified. That is, for example, the weighting processing unit 12 calculates, for a partial base composition at the 5'-terminal position or the 3'-terminal position, a first predicted mass value obtained without considering the presence of a predetermined functional group added to a terminal base, and a second predicted mass value obtained considering the presence of the functional group, and performs matching processing for those predicted mass values with an actual mass value. When the second predicted mass value exhibits higher coincidence with the actual mass value, the analysis processing unit 13 may determine that the functional group has been added to a base at the 5'-terminus or the 3'-terminus. Then, the analysis processing unit 13 creates such a directed graph that a modified base is displayed in a distinguishable manner from the other bases, for example. That is, when a base forming a difference in partial base composition between nodes selected in each hierarchy is displayed in a corresponding manner to an edge for connecting the nodes, a modified base in the base forming a difference is displayed by linking a character and a symbol indicating that a modification has been made to the modified base. The analysis processing unit 13 outputs directed graph data for drawing such a directed graph to the output unit 40, and the output unit 40 presents an interface screen having the directed graph displayed to a user. A user to whom such a directed graph has been presented may visually understand not only analysis results of a base sequence but also analysis results of a modification structure.

As described above, according to the present invention, a base modification structure contained in a nucleic acid may be analyzed quickly and precisely. That is, according to the present invention, qualitative information such as posttranscriptional processing and a modification structure may also be obtained for an RNA molecule, for example.

Further, in the method of the present invention, selection processing for selecting one of base composition sets belonging to each hierarchy while following connection relations from the outermost hierarchy in a hierarchical structure based on a weight imparted to each of base composition sets may be performed from the outermost hierarchy to any internal hierarchy. That is, in this case, the analysis processing unit 13 receives instruction data for designating a hierarchy depth at which selection processing is carried out via the input unit 30 from a user, for example. Then, the analysis processing unit 13 sequentially carries out selection processing from the outermost hierarchy to the internal hierarchy corresponding to the hierarchy depth designated by the instruction data. As a result, the analysis processing unit 13 may determine a base sequence and a base modification for a part on the terminal side (a portion from the terminus to the position corresponding to the designated hierarchy depth) of the target fragment.

Specifically, for example, when the base composition processing unit 11 creates a hierarchical structure consisting of nine hierarchies, and the analysis processing unit 13 receives instruction data for designating, as an internal hierarchy at which selection processing is terminated, the third hierarchy from the outermost hierarchy, the analysis processing unit 13 determines the third hierarchy in the third from the 5'-terminus as a first central hierarchy, and determines the seventh hierarchy in the third from the 3'-terminus as a second central hierarchy. Then, the analysis processing unit 13 terminates selection processing when selecting one of base composition sets belonging to each hierarchy from the outermost hierarchy (first hierarchy) at the 5'-terminus to the first central hierarchy (third hierarchy) and from the outermost hierarchy (ninth hierarchy) at the 3'-terminus to the second central hierarchy (seventh hierarchy), respectively. It should be noted, in this case, the weighting processing unit 12 may also terminate weight update processing when updating a weight of each node from the first hierarchy at the 5'-terminus to the third hierarchy, and from the ninth hierarchy at the 3'-terminus to the seventh hierarchy, respectively. Then, as mentioned above, the analysis processing unit 13 compares corresponding partial base sequences among base composition sets selected in each of those hierarchies as targets of selection processing, and specifies a base forming a difference between corresponding partial base compositions. Thus, the analysis processing unit 13 may determine a base sequence and a base modification for a part on the 5'-terminal side (a portion from the 5'-terminus to the position corresponding to the first central hierarchy) of the target fragment, and a part on the 3'-terminal side (a portion from the 3'-terminus to the position corresponding to the second central hierarchy) of the target fragment.

Further, in this case, the output unit 40 may first present information helpful for determining a hierarchy depth at which selection processing is performed to a user. That is, for example, the output unit 40 displays, on a display device, an interface image representing reference information including information relating to mass spectrometry results of a parent ion such as a molecular weight and a terminal structure (e.g., the kind of a functional group added to a terminal base) of a target fragment, information relating to mass spectrometry results of product ions such as a mass spectrum observed for product ions, and information relating to the results of matching processing for a theoretical spectrum and an actual spectrum. As a result, the user may determine, based on the reference information represented on the interface image, either performing selection processing for all hierarchies contained in a hierarchical structure or performing selection processing for a part of hierarchies on the terminal side. In addition, in the case of performing selection processing for only one part of hierarchies, the user may determine a hierarchy range in which selection processing should be performed. Then, the input unit 30 receives, from the user, instruction data for designating a hierarchy range in which selection processing should be performed.

As described above, selection processing for only any one part of hierarchies at the terminus in a hierarchical structure allows a base sequence and a base modification to be determined quickly and precisely for at least one part on the terminal side of a nucleic acid while an amount of calculation is reduced, even if a nucleic acid of interest has a long molecular chain, and mass spectrometer performance and measurement conditions (such as a nucleic acid sample amount and a condition for dissociating a parent ion) used for measurement of product ions are insufficient, and thus reliable weighting is not performed for base composition sets classified into around the center of the hierarchical structure, for example.

Further, as described above, when a base sequence has been determined only for one part on the terminal side of a nucleic acid, the analysis processing unit 13 may perform, based on, for example, the one part of base sequences and the total base composition of the nucleic acid, search processing for a database including registered known nucleic acid base sequences, and determine the total base sequence of the nucleic acid based on the results of the search processing. That is, for example, when a human RNA fragment is used as an analytic target, the analysis processing unit 13 searches, out of a human genome based on a human genome database, a genome portion satisfying the search condition that its base composition corresponds to the total base composition of the RNA fragment, and its base sequence at the terminal portion corresponds to the determined base sequence of the RNA fragment. Then, the analysis processing unit 13 determines the total base sequence of the RNA fragment based on the base sequence of the genome portion, and specifies the position thereof on the human genome, in the case of detecting the genome portion satisfying the search condition.

It should be noted that, as described above, the analysis processing unit 13 may utilize, for example, an RNA mass finger printing method (RMF) as a method of performing search processing for a database including registered known nucleic acid base sequences. That is, when a target fragment is a human RNA fragment prepared by cleaving human RNA with a ribonuclease (for example, RNase T1), the analysis processing unit 13 first creates a virtual genome fragment by virtually cleaving the position (e.g., the position of guanine (G) in the case of using RNase T1) at which the ribonuclease specifically cleaves a human genome based on a human genome database. That is, the analysis processing unit 13 produces, for example, a virtual fragment database linking each virtual genome fragment to a position of each virtual genome fragment on a human genome. Further, the analysis processing unit 13 determines the total base sequence of the target fragment based on a molecular weight of a parent ion obtained in mass spectrometry of the parent ion derived from the target fragment. In addition, the analysis processing unit 13 determines a base sequence in the vicinity of the terminus of the target fragment as mentioned above. Further, the analysis processing unit 13 determines a frame length corresponding to the full length of human RNA used for preparing the target fragment based on, for example, instruction data received via the input unit 30 from a user. Then, the analysis processing unit 13 carries out the following search processing for a human genome based on the human genome database, the virtual fragment database, the total base composition of the target fragment, the base sequence in the vicinity of at the terminus of a target fragment, and the frame length. That is, the analysis processing unit 13 determines, as a search target, a partial region having a length of the above-mentioned frame length out of a human genome, and searches whether or not the partial region contains a virtual genome fragment satisfying the condition that its base composition corresponds to the total base composition of the target fragment determined as mentioned above, and its base sequence at the terminal portion corresponds to the base sequence of the target fragment determined as mentioned above. The analysis processing unit 13 sequentially determines, as search targets, partial regions that are different from each other on a human genome, while performing scanning in a frame length on the human genome, and carries out the above-mentioned search processing for each of partial regions as the search targets. Then, the analysis processing unit 13 judges that the virtual genome fragment has a hit on the human genome when at least one virtual genome fragment contained in each of partial regions satisfies the above-mentioned condition. In this case, the analysis processing unit 13 imparts a high score to a virtual genome fragment having a small hit frequency (occurrence frequency) in the human genome out of the hit virtual genome fragments, and also imparts the score to a partial region containing the virtual genome fragment. It should be noted that the score may be calculated from, for example, occurrence probability of a virtual genome fragment calculated based on binomial theorem. Then, the analysis processing unit 13 ranks the partial regions based on their scores, and determines, based on a partial region with a high score, a position on the human genome of human RNA used for preparing the target fragment, and determines the total base sequence of the human RNA (including the total base sequence of the target fragment in part). That is, the analysis processing unit 13 determines, for example, a partial region with the highest score as a genome portion corresponding to human RNA, and determines, based on a base sequence of the partial region, the total base sequence of human RNA containing the target fragment.

Next, there is described a specific example where the method of the present invention has been performed by using the apparatus of the present invention that operates in accordance with the program of the present invention.

### [Example 1]

An RNA fragment group was prepared by allowing RNase A to act on a purified *Escherichia coli* 5S ribosomal RNA molecule. The RNA fragment group was measured with an LC/MS mass spectrometer (LC-QqTOF) (QStar, Applied Biosystems Japan Ltd.). It should be noted that this mass spectrometer was used in all of the following examples. Ionization was performed by an ESI method. From the measurement data, an ion having an m/z value of "999. 7" and a valence of "-2" was selected as a parent ion of an RNA fragment as an analytic target. Then, the parent ion was dissociated by CID in the mass spectrometer to obtain an MS/MS mass spectrum. The collision energy in CID was 45 eV. The total base composition determined based on the m/z value and the valence of the parent ion was "A3UG2". In addition, the directed graph as shown in FIG. 6 was created based on the total base composition.

Further, with respect to each of nodes contained in the directed graph, a theoretical spectrum was obtained by calculating a theoretical m/z value of each of a, b, c, d, w, x, y, and z series of product ions corresponding to each of partial base compositions. That is, a predicted m/z value was theoretically calculated from a partial base composition at the 5'-terminal position for a, b, c, and d series of product ions derived from the 5'-terminal portion, and from a partial base composition at the 3'-terminal position for w, x, y, and z series of product ions derived from the 3'-terminal portion, respectively.

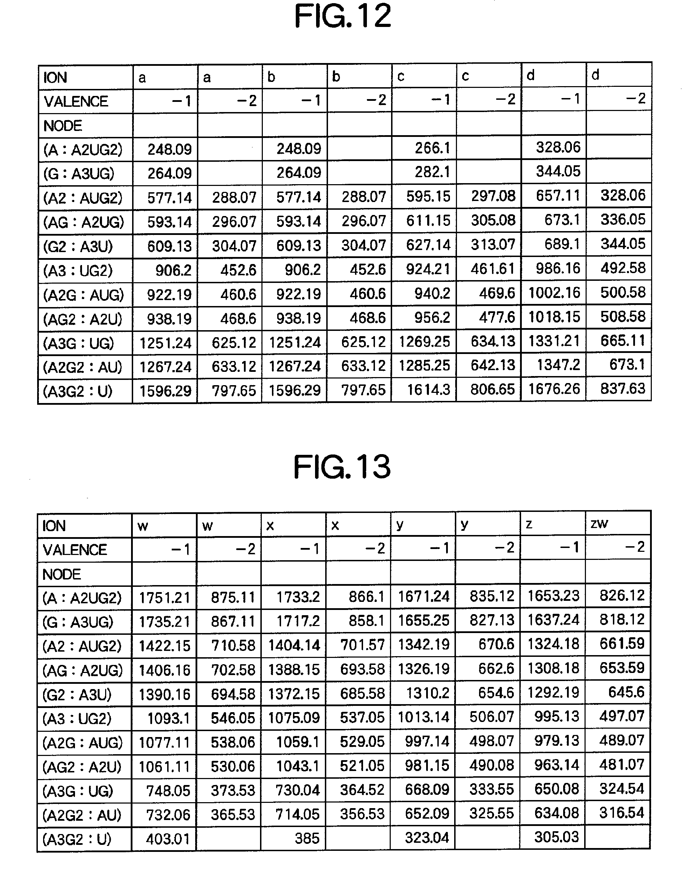

FIGS. 12 and 13 are each a data table illustrating predicted m/z values which have been theoretically calculated. In those data tables, each base composition set is linked to predicted m/z values of product ions which may be produced in a corresponding manner to a partial base composition at the 5'-terminal position or the 3'-terminal position of each base composition set. That is, the structure of each base composition set, the series and the valence of each product ion, and the predicted m/z value of each product ion are linked to each other. It should be noted that when an m/z value is calculated from a molecular weight of a product ion, the calculation was performed by using a value ranging from "-1" to "-2" as a valence of a parent ion, provided that, for a product ion having a base sequence length of "1" (product ion consisting of one base), the calculation was performed by using only "-1" as a valence.

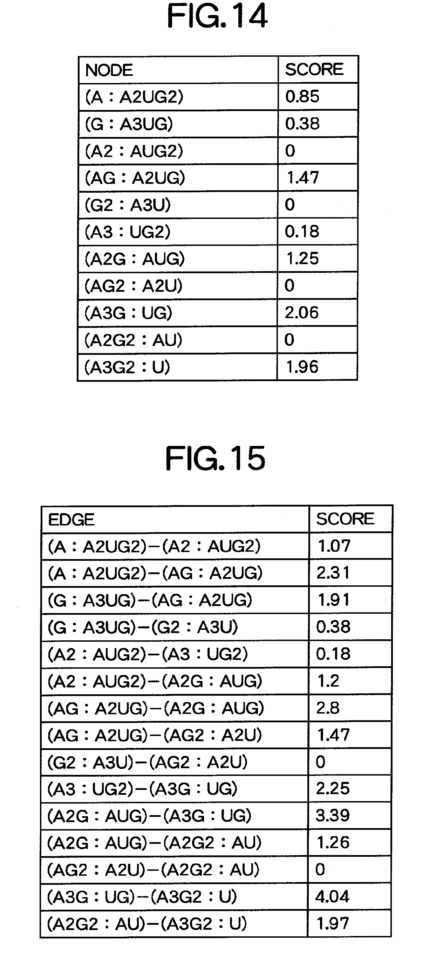

Then, the tolerance of the mass spectrometer was set to "0.2", and those theoretical spectra were matched with actual mass spectra. When a difference between a predicted m/z value and an actual m/z value falls within "0.2", the predicted m/z value and the actual m/z value were judged as being coincident with each other. Then, when the predicted m/z value in the theoretical spectrum was matched with the actual m/z value in the actual spectrum, the total of the intensity of matched observed peaks was used as a score (weight) of a node. For example, in a node corresponding to a base composition set having a structure of "AG:A2UG", theoretical spectra corresponding to a, b, c, and d series and w, y, and z series of product ions were matched with a part of actual spectra. Thus, the total of the intensity of those matched peaks was used as a score for the node. Based on the results, the data table linking each node to a score as shown in FIG. 14 was created.

Next, an edge for connecting nodes was scored. The total of scores for a pair of nodes connected to both termini of the edge was used as a score for the edge. Based on the results, the data table linking each edge to a score as shown in FIG. 15 was created.

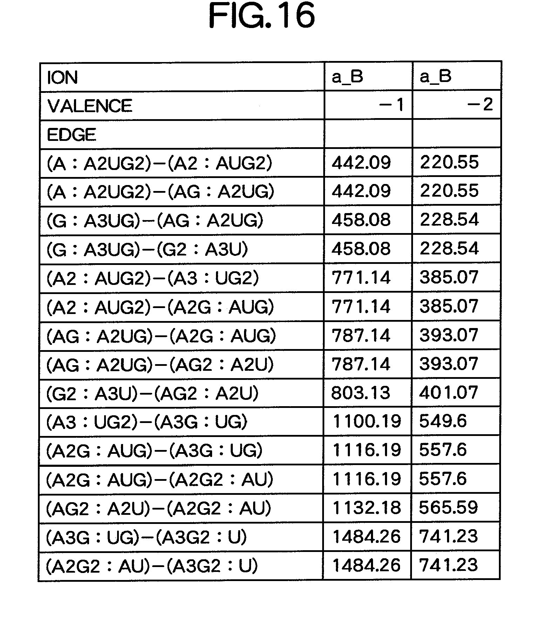

In addition, for an "a_B" ion lack of a terminal base, predicted m/z values of the "a_B" ion were calculated from information on an edge (i.e., the structure of base composition sets corresponding to two nodes to be connected with an edge). When the predicted m/z value was matched with an actual spectrum, the intensity of the matched peak was added to a score for the edge. For example, from an edge with which a node having a structure of "A:A2UG2" to a node having a structure "AG:A2UG" are connected to each other, a predicted m/z value of an "a_B" ion in which a base portion of "G" has been deleted from a composition of "AG" was calculated as "442.09". FIG. 16 is a data table linking each edge to a predicted m/z value of a base deficient ion corresponding to each edge. In this example, because the predicted m/z value was also hit at the actual spectrum, the intensity of the hit peak was also added to a score for an edge.

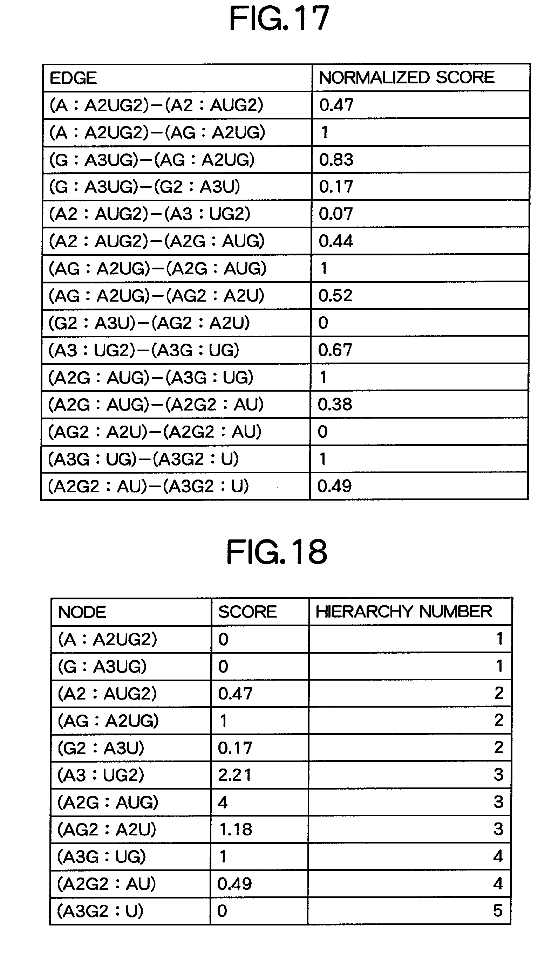

In addition, the score for an edge was normalized. That is, a score for each edge in each hierarchy was divided by the highest score among scores imparted to edges in each hierarchy. FIG. 17 is a data table linking each edge to a normalized score.

Then, update processing for a score for each node was performed from nodes at both termini by applying dynamic programming. FIG. 18 is a data table linking each node, an updated score, and a hierarchy number to which each node belongs after the update processing for a score, to each other.

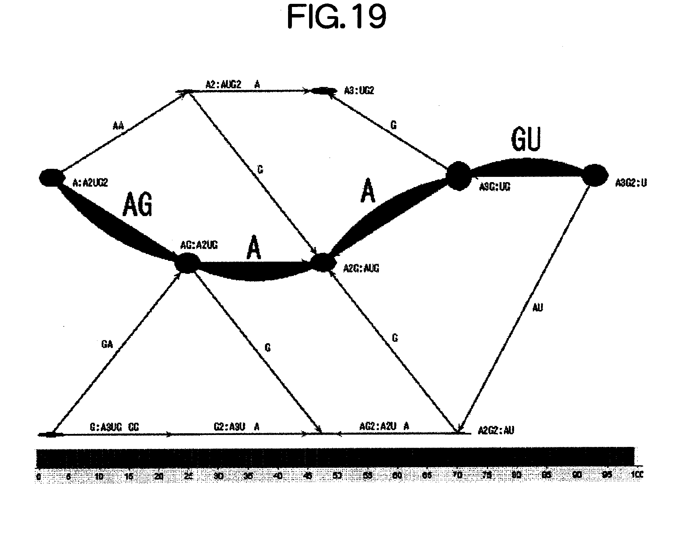

As a result, the directed graph as shown in FIG. 19 was created. As shown in FIG. 19, in the central hierarchy, a node corresponding to the base composition set "A2G:AUG" was selected as a node having the highest score. In addition, trace back processing was performed in accordance with the program of the present invention, and as nodes arranged on an optimum route, the node "AG:A2UG" in the second hierarchy and the node "A:A2UG2" in the first hierarchy at the 5'-terminus were selected in the stated order from the node "A2G:AUG" selected in the third hierarchy as the central hierarchy toward the 5'-terminal side, and the node "A3G: UG" in the fourth hierarchy and the node "A3G2:U" in the fifth hierarchy at the 3'-terminus were selected in the stated order from the node "A2G:AUG" in the central hierarchy toward the 3'-terminal side, respectively. As a result, the total base sequence of the RNA fragment of interest was determined as "AGAAGU", which coincided with a correct total base sequence.

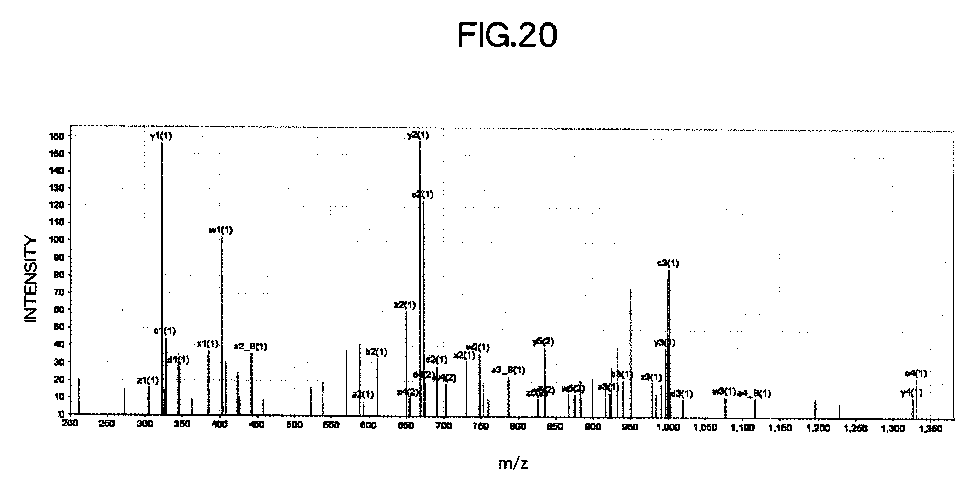

It should be noted that, in the directed graph after the determination of an optimum route as shown in FIG. 19, the size of each node with a circular shape or an oval shape reflects a relative score to other nodes in a hierarchy to which each node belongs, and a node with a higher score is displayed in a larger size. Further, the thickness of the edge reflects the level of a score imparted to the edge, and an edge with a higher score is displayed in a larger thickness. In addition, in the directed graph as shown in FIG. 19, each node and each edge may also be displayed with colors depending on imparted scores. The bar as shown in the bottom portion of FIG. 19 is a scale bar representing the gradation of the colors. Further, FIG. 20 illustrates an MS/MS mass spectrum by labeling peaks which have been hit at a theoretical spectrum corresponding to nodes forming an optimum route, out of peaks of observed product ions. That is, the labeled peaks are peaks whose actual m/z values match with the predicted m/z values.

### [Example 2]

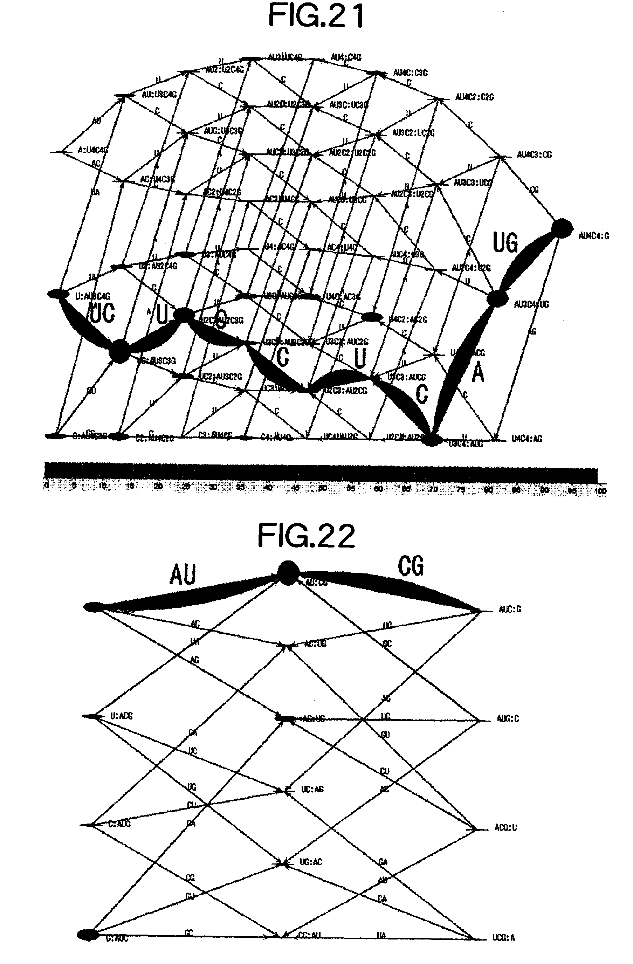

*Escherichia coli* 5s RNA similar to that in Example 1 was cleaved with RNase T1 and the obtained RNA fragment was analyzed for its base sequence. An RNA fragment corresponding to a parent ion having an m/z value of "1044.5" and a valence of "-3" was used as an analytic target. The total base composition of the RNA fragment was calculated as "AU4C4G". The collision energy used for CID in producing product ions from the parent ion was 50 eV. In this example, a score for an edge was not normalized. As a result of the analysis, the total base sequence of the RNA fragment was determined as "UCUCCUCAUG", which coincides with an actual total base sequence of the RNA fragment. FIG. 21 illustrates a directed graph after the determination of an optimum route in this example.

### [Example 3]

A base sequence was analyzed with the algorithm of the present invention by using a synthetic RNA having a total base sequence of "AUCG". The parent ion had an m/z value of "1222.2" and a valence of "-1". The total base composition was determined as "AUCG". The collision energy in CID was 50 eV. The terminal base could not be preliminarily determined because an RNase cleavage fragment was not used, but the total base sequence could be determined. FIG. 22 illustrates a directed graph after the determination of an optimum route.

### [Example 4]

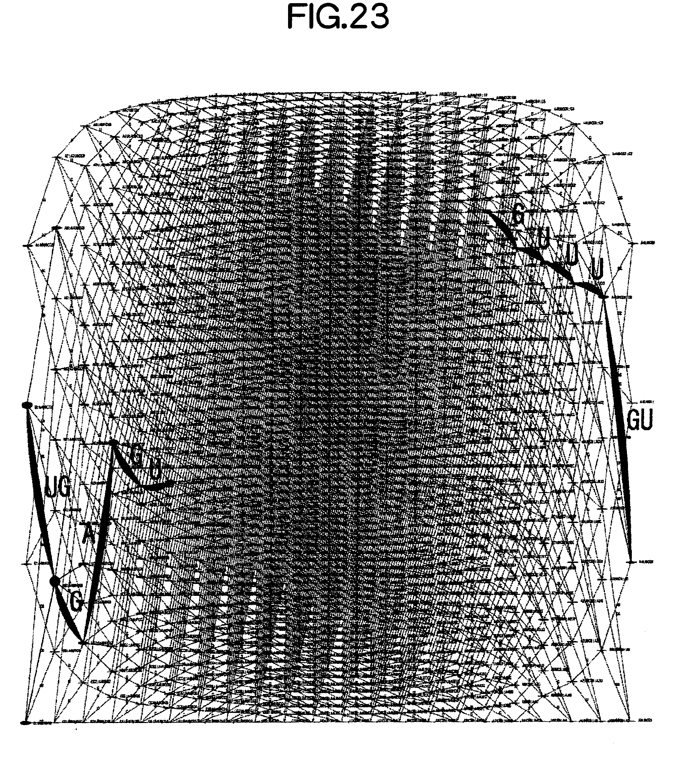

A synthetic micro RNA (mmu-miR-122a) having a base sequence of "UGGAGUGUGACAAUGGUGUUUGU" was used as an analytic target, and the base sequence was analyzed only for a portion having a predetermined desired length from both termini of the micro RNA. That is, in this example, the apparatus 1 of the present invention received, via the input unit 30 from a user, instruction data for designating the number of bases at the terminal portion as an analytic target as "6", as instruction data for designating a hierarchy depth as an analytic target. Then, based on the instruction data, the control unit 10 in the apparatus 1 of the present invention analyzed a node having a partial base composition at the 5'-terminal position having 1 to 6 bases, and a node having a partial base composition at the 3'-terminal position having 1 to 6 bases, as targets. The parent ion had an m/z value of "832.2" and a valence of "-9". The total base composition was determined as "A4U9CG9". The collision energy in CID was 25 eV. The apparatus 1 of the present invention carried out route search based on dynamic programming until the sixth hierarchy from each of the 5'-terminus and the 3'-terminus in a directed graph. Based on the results of the route search, a partial base sequence from the 5'-terminus to the sixth residual portion of a micro RNA and a partial base sequence from the 3'-terminus to the sixth residual portion could be exactly determined as "5'UGGAGU" and "GUUUGU3"', respectively. FIG. 23 illustrates a directed graph after the determination of an optimum route.

### [Example 5]

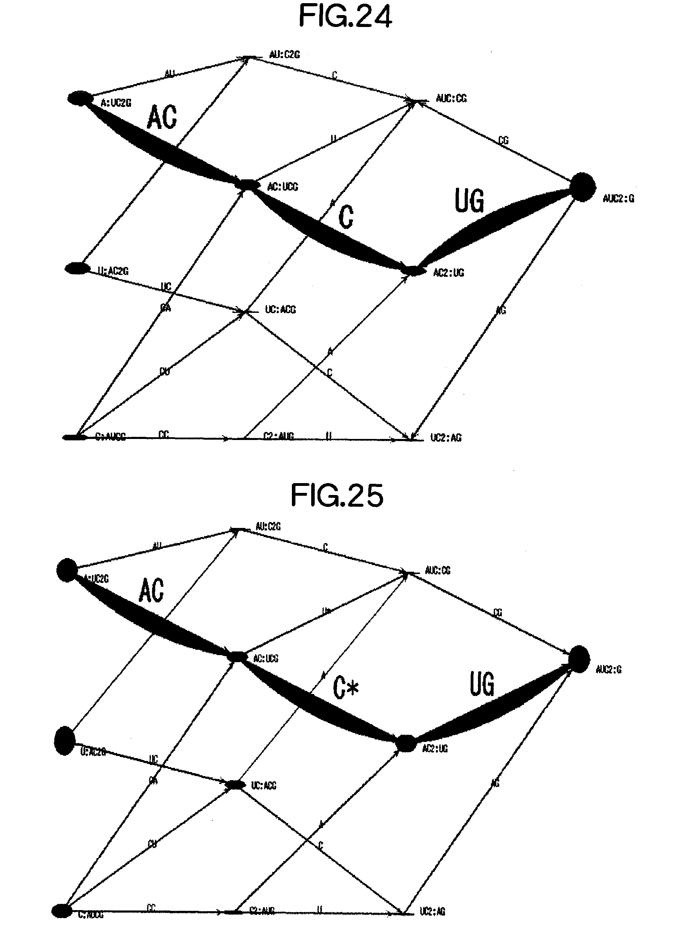

An RNA fragment prepared using RNase T1 was used as an analytic target. The parent ion had an m/z value of "986.2" and a valence of "-2". Analysis was performed in the case of taking the presence of a modification group into consideration and in the case of not doing so. FIG. 24 illustrates a directed graph finally obtained in the case of not taking the presence of a modified base into consideration. Also in this case, the total base sequence of the RNA fragment could be exactly determined as "ACCUG".

Further, FIG. 25 illustrates a directed graph in the case of determining the total base sequence and the position of a modified base by using a predicted m/z value calculated by correction in taking the presence of a modified base into consideration. In this case, the total base sequence of the RNA fragment could be exactly determined as "ACCUG", and further, the position of a base to which a methyl group had been added as a modification group could be identified. That is, in the directed graph as shown in FIG. 25, because a predicted m/z value of the partial base composition "UCG" at the 3'-terminal position of the node "AC:UCG" and a predicted m/z value obtained by correcting a molecular weight corresponding to a methyl group to the base composition "AC2" at the 5'-terminal position of the node "AC2 : UG" were highly matched with (had a smaller difference in m/z value from) the actual m/z value compared with a predicted m/z value without correction, it was judged that a base modification with a methyl group existed on the central cytosine (C) in the total base sequence "ACCUG". That is, it could be precisely detected that, in the RNA fragment having a base sequence of "ACCUG", the third cytosine (C) from the 5'-terminus was modified with a methyl group. It should be noted that, in FIG. 25, the mark "*" imparted to cytosine (C) indicates that the cytosine (C) base has been modified. Further, as may be easily recognized by a difference in size between two nodes, stronger peak intensity was detected for the two nodes containing the above-mentioned modified base in FIG. 25 compared with FIG. 24.

### [Example 6]

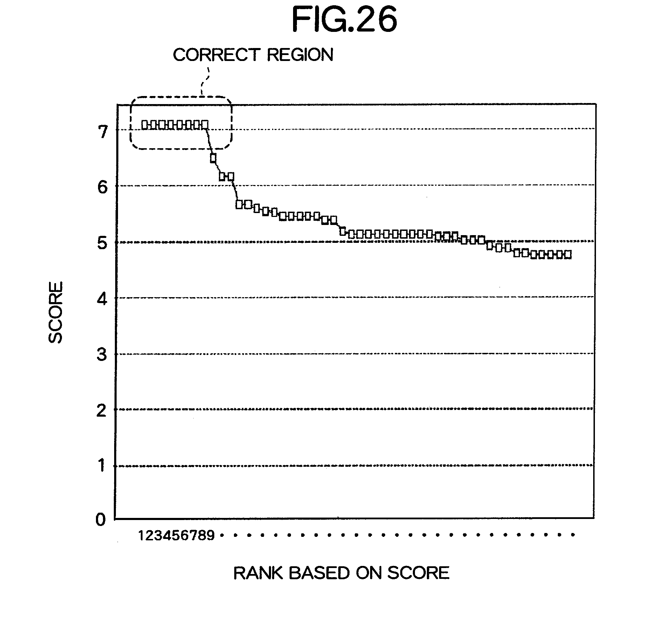

This is an example where an RNA fragment group was prepared by digesting *Escherichia coli* 5s RNA with RNase T1, seven RNA fragments in the RNA fragment group were measured with an LC-QqTOF type mass spectrometer, and the position of a gene corresponding to the 5s RNA was precisely detected by an RNA Mass Finger printing (RMF) method based on the total base composition of each of the RNA fragments, from an *Escherichia coli* genome database. That is, in a corresponding manner to seven RNA fragments, seven parent ions having molecular weights of "2829.43", "2547.35", "2266.32", "1655.25", "1632.23", "1302.19", and "1279.16" were measured by mass spectrometry. Then, the total base composition of RNA fragments corresponding to seven parent ions was determined as "AU2C5G", "A2UC4G", "A3UC2G", "A3CG", "A2UCG", "A1C2G", and "UC2G", respectively. Then, for the *Escherichia coli* genome database, processing for searching a genome partial region having a frame length corresponding to the length of 5s RNA, the genome partial region containing portions whose base compositions are the above-mentioned seven total base compositions, was carried out to obtain the results as shown in FIG. 26. That is, as shown in FIG. 26, the top eight partial regions to each of which the same highest score has been imparted, the partial regions being contained in an *Escherichia coli* genome and being surrounded by a dashed line in the figure, were detected as correct regions. It should be noted that when each of copies of the same sequence exists at a plurality of positions on the genome, a plurality of partial regions with the same score may be detected as mentioned above.

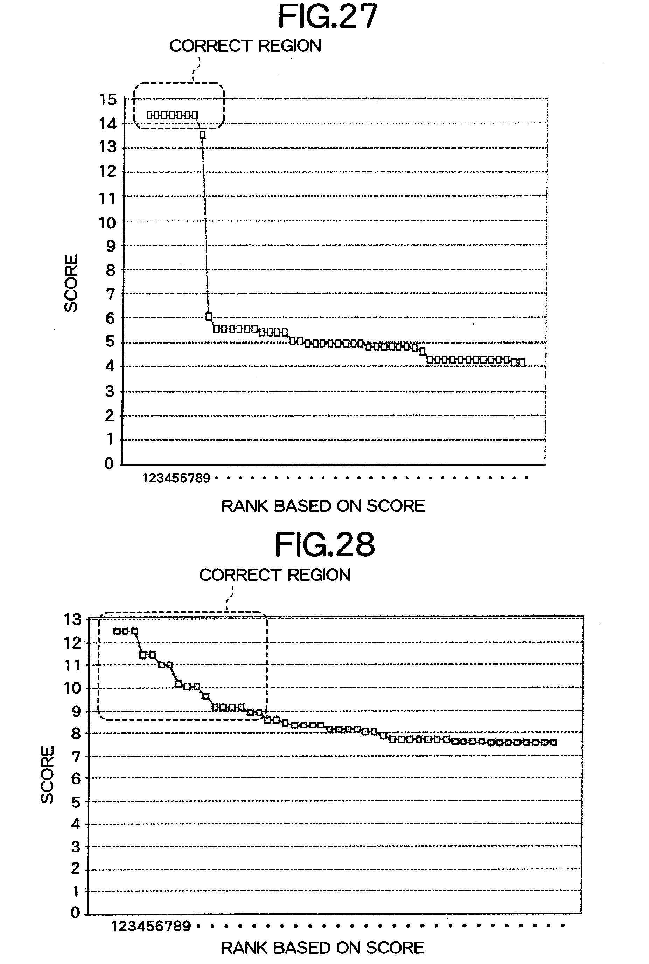

Meanwhile, the above-mentioned method for analysis according to the present invention could be applied to those RNA fragments, to thereby determine the total base sequence of seven RNA fragments as "UCCCACCUG", "ACCCCAUG", "AACUCAG", "AAACG", "AACUG", "CCAG", and "CCUG", respectively. Then, with the use of an RMF method as a part of the method for analysis according to the present invention, processing for searching a genome partial region with a frame length corresponding to the length of 5s RNA, the genome partial region containing the above-mentioned seven base sequences was carried out for the *Escherichia coli* genome database, to thereby obtain the results as shown in FIG. 27. That is, as shown in FIG. 27, the detection sensitivity of the RMF method could be enhanced by carrying out search to which the information relating to the base sequence determined by the present invention has been added. In FIG. 27, the top seven partial regions surrounded by a dashed line in the figure out of the top eight partial regions in FIG. 26 could be further extracted as correct regions with the highest score. Further, a difference in score between those eight partial regions and the other partial regions could be remarkably increased compared with the case as shown in FIG. 26, and correct candidates and incorrect candidates could be more clearly separated from each other.

### [Example 7]

This is an example where human 7sk RNA was searched by incorporating the algorithm of the present invention into the RMF method. A ribonucleoprotein (RNP) containing human 7sk RNA was immunoprecipitated with an antibody, and the immunoprecipitate was purified to afford a sample containing the human 7sk RNA. The sample was subjected to in-gel fragmentation treatment with RNase T1, and a sample after the fragmentation treatment was analyzed with an LC/MS mass spectrometer. As a result, six RNA fragments had molecular weights of "3464.5", "2805.4", "2572.4", "2548.3", "2526.3", and "2500.3" (Da), respectively. Of those, two RNA fragments having molecular weights of "3464.5" and "2548.3" were analyzed with the algorithm of the present invention. As a result, the total base sequence of the two RNA fragments could be determined as "UCACCCCAUUG" and CUUCCAAG", respectively. Then, with the use of the RMF method, in a 300-base width (frame length) corresponding to the length of the human 7sk RNA, a genome portion having the total base composition of the human 7sk RNA, and a partial base sequence (the total base sequence of the above-mentioned two RNA fragments) of the determined human 7sk RNA was searched on a human genome. As a result, as shown in FIG. 28, all of the top 17 genome portions to which high scores have been imparted were hit at a human 7sk gene. Meanwhile, database search was performed in the same manner using only the total base composition without using information on a partial base sequence. The results revealed that the genome portions could not be sufficiently differentiated by scores to determine a correct base sequence.

It should be noted that the present invention is not limited to the above-mentioned examples. That is, a nucleic acid as an analytic target is not limited to RNA and an RNA fragment, and any nucleic acid such as DNA, a DNA fragment prepared with a DNase, and further, various modified nucleic acids synthesized for nucleic pharmaceuticals may be used as an analytic target. Further, the length of the nucleic acid as an analytic target is not particularly limited. In determining the total base sequence of the nucleic acid without utilizing database search of known sequences, for example, a nucleic acid having 20 or less bases (such as micro RNA (miRNA)) is preferably used as a target, and a nucleic acid having 15 or less bases is particularly preferably used as a target. It should be noted that the preferred number of bases may be increased if the analysis accuracy of the mass spectrometer (also including the ionization efficiency of the nucleic acid) is improved, and does not limit an analytic target according to the present invention. Further, with the use of mass spectrometry with not only a mass spectrometer (MS/MS) including two mass separation units but also, for example, a mass spectrometer (MSⁿ) including three or more mass spectrometry units, a nucleic acid having a larger number of bases may also be determined for its total base sequence. That is, in this case, for example, by using a mass spectrometer (MS/MS/MS) including three mass spectrometry units, a parent ion is measured by first mass spectrometry (MS), a product ion (daughter ion) of the parent ion is measured (MS/MS) by second mass spectrometry, and a product ion (granddaughter ion) of the daughter ion is measured by third mass spectrometry (MS/MS/MS). The mass spectrometry results of the granddaughter ion may be utilized to determine the total base sequence of the daughter ion, and the mass spectrometry results of the daughter ion and the total base sequence may be utilized to determine the total base sequence of a nucleic acid of interest. Further, the total base composition used for creating base composition sets is not limited to one determined based on the actual mass spectrometry results of the parent ion, and for example, may also be any total base composition input by a user.

Further, the order of processing included in the method of the present invention may be appropriately set in view of processing efficiency and the like, and a part of the above-mentioned processing may be carried out in parallel in a distinguishable manner from each other. That is, for example, the control unit 10 in the apparatus 1 of the present invention may sequentially or concurrently carry out, based on the program of the present invention, processing for creating base composition sets, processing for creating a hierarchical structure of base composition sets, and processing for creating connection relations between base composition sets. Specifically, for example, when a target fragment contains "n" bases, a terminal base of the target fragment cannot be specified with the kind of an RNase and etc., and a base composition set consisting of a pair of partial base compositions is created, with the base composition processing unit 11 first reading out, from the memory unit 20, a set generating function for generating an object which holds a base composition set containing "1" base in a partial base composition at the 5'-terminal position and containing "n-1" base (s) in a partial base composition at the 3'-terminal position. Further, a base composition processing unit 11 determines, as the terminal base, a base contained in the total base composition out of adenine (A), uracil (U), cytosine (C), and guanine (G). Then, the base composition processing unit 11 produces, based on the set generating function, an object linking a base composition set having a partial base composition at the 5'-terminal position consisting of the one terminal base and having a partial base composition at the 3'-terminal position of a base composition in which the terminal base has been removed from the total base composition, to the hierarchy number "1" of the outermost hierarchy having the smallest number of bases in a partial base composition at the 5'-terminal position, and stores the object in the memory unit 20. Similarly, in creating a base composition set containing "i" bases in a partial base composition at the 5'-terminal position ("i" represents an integer of 2 to n) and containing "n-i" bases in a partial base composition at the 3'-terminal position, the base composition processing unit 11 first calls up all of base composition sets, in each of which the number of bases in a partial base composition at the 5'-terminal position is smaller by one than above, the sets having been linked to the hierarchy number "i-1" smaller by one than above, and takes out a partial base composition at the 5'-terminal position and a partial base composition at the 3'-position forming each of the called base composition sets. In addition, for each of base composition sets linked to the hierarchy number "i-1", the base composition processing unit 11 determines, as a movement base, a base contained in a partial base composition at the 3'-terminal position out of A, U, C, and G. Then, the base composition processing unit 11 newly produces, based on a set generating function, an object linking a base composition set consisting of a partial base composition at the 5'-terminal position obtained by adding one movement base to a partial base composition at the 5'-terminal position of each base composition set which has been linked to the hierarchy number "i-1", and a partial base composition at the 3'-terminal position obtained by removing one movement base from a partial base composition at the 3'-terminal position of each base composition, to the hierarchy number "i" corresponding to the number of bases in a partial base composition at the 5'-terminal position, and stores the object in the memory unit 20. Further, the base composition processing unit 11 produces an object holding a connection relation between an object of a base composition set linked to the hierarchy number "i-1" and an object of a base composition set linked to the hierarchy number "i", and stores the obj ect in the memory unit 20. That is, the base composition processing unit 11 produces connection data representing a connection relation between each base composition set linked to the hierarchy number "i-1", and a base composition set having a partial base composition at the 5'-terminal position obtained by adding one movement base to a partial base composition at the 5'-terminal position of each base composition set, out of composition sets linked to the hierarchy number "i", and stores the connection data in the memory unit 20. It should be noted that, in producing the connection relation, when a partial base composition at the 5'-terminal position of a new base composition set to be connected with the newly created connection relation coincides with a partial base composition at the 5'-terminal position in the base composition set in the object which has been already stored, the base composition processing unit 11 does not store an obj ect of the new base composition set, but produces an object holding a new connection relation with an object of the already stored base composition set, and stores the resultant object in the memory unit 20. That is, the base composition processing unit 11 produces and stores one object for one set of the partial base compositions. The base composition processing unit 11 carries out such processing for "i" of "1" to "n".

Further, the weight to be imparted to base composition sets and nodes is not limited to the peak intensity of a product ion, and may be a weight to be calculated with a predetermined function depending on the peak intensity, for example. Further, for example, the weight may be determined with an error between a predicted mass value for a partial base composition and an actual mass value for each product ion and the number of actual peaks hit at a theoretical spectrum predicted for each partial base composition, in addition to each peak intensity. That is, for example, the weight may be larger as the difference between the predicted mass value and the actual mass value is smaller. Further, for example, based on a dissociation condition (such as a dissociation energy in CID) of a parent ion and the like, a mass value corresponding to a major peak which is expected to be measured at the intensity equal to or more than a predetermined threshold out of a plurality of mass values (mass values different from each other to be calculated from series that are different from each other such as c-y series and a-w series) predicted for each partial base composition is determined as a focused mass value, and in the actual spectrum, a weight may also be determined based on the number of peaks detected (i.e., the number of the hit major peaks) at the position of an actual mass value coincident with the focused mass value.

Further, search for an optimum route in the directed graph is not necessarily performed by dynamic programming, and may also be performed by, for example, a greedy algorithm and other route searching methods. That is, for example, when the greedy algorithm is applied, the analysis processing unit 13 may carry out selection processing for selecting a node to which the largest weight has been imparted from nodes belonging to each hierarchy sequentially from at least one outermost hierarchy, to thereby determine a base sequence sequentially from at least one terminal base of a nucleic acid.

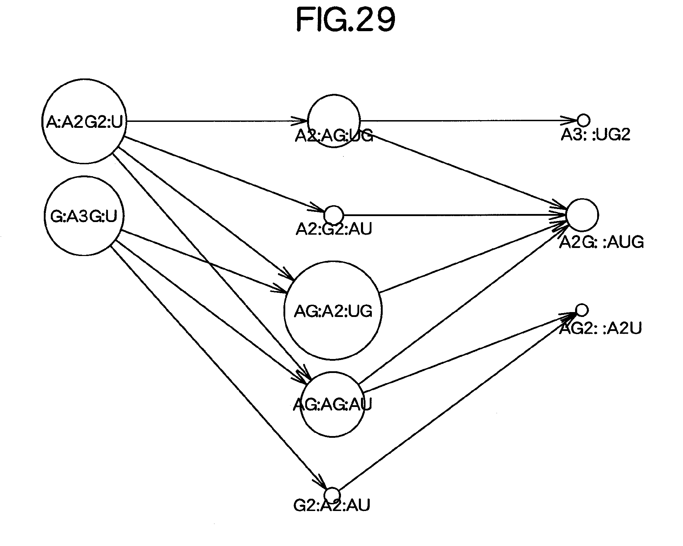

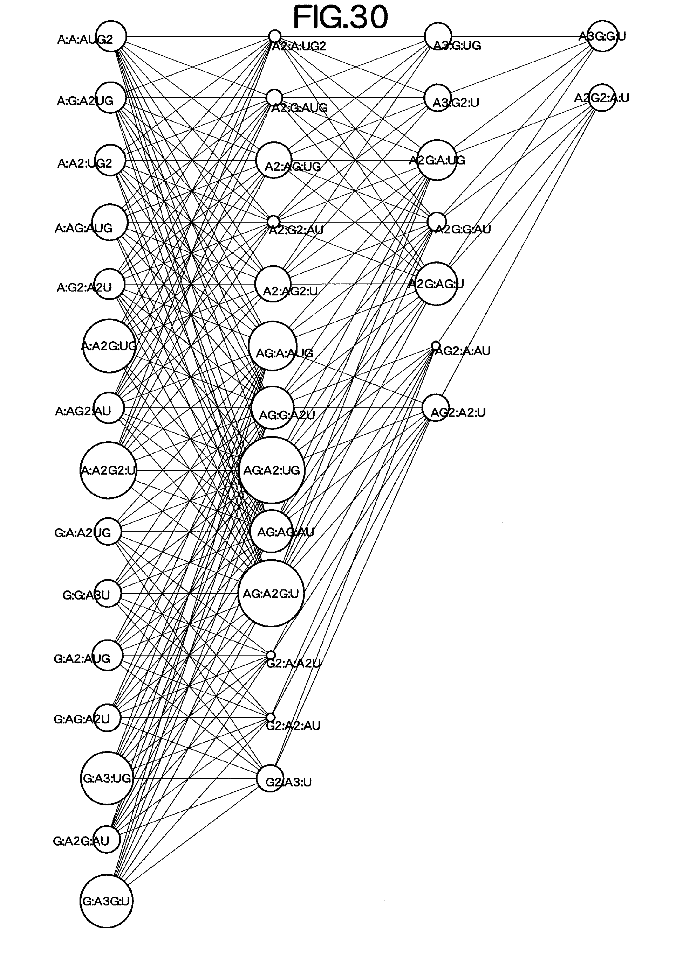

Further, the base composition set is not limited to one consisting of a set of partial base compositions obtained by dividing the total base composition into two, and may be one consisting of a set of partial base compositions obtained by dividing the total base composition into three or more. That is, in the case of creating a base composition set consisting of a set of partial base compositions obtained by dividing the total base composition into three, the directed graphs as shown in FIGS. 29 and 30 may be created, for example.

In the example as shown in FIG. 29, base composition sets are hierarchized in ascending order of the number of bases in partial base compositions at both the terminal positions. That is, in this example, in a base composition set corresponding to each node, the number of bases in a partial base composition at the 5'-terminal position and the number of bases in a partial base composition at the 3'-terminal position are identical to each other, and base composition sets are hierarchized so that the number of bases in both the partial base composition at the 5'-terminal position and the partial base composition at the 3'-terminal position will be increased one by one from the 5'-terminus to the 3'-terminus in the directed graph. In this case, selectionprocessing for selecting one of nodes contained in each hierarchy from the 5'-terminus to the 3'-terminus in the directed graph allows a base sequence to be sequentially determined from both terminal bases of a nucleic acid as an analytic target.

Further, in the example as shown in FIG. 30, base composition sets are hierarchized in ascending order of the number of bases in a partial base composition at the 5'-terminal position as one terminal position. That is, in this example, selection processing for selecting one of nodes contained in each hierarchy from the 5'-terminus to the 3'-terminus in the directed graph allows a base sequence to be determined sequentially from the 5'-terminal base of a nucleic acid as an analytic target.

Further, the apparatus 1 of the present invention does not necessarily include the mass spectrometry unit 50 in a part of the structure. That is, for example, the apparatus 1 of the present invention may be installed in an analysis room physically separated from another analysis room installed with a mass spectrometer. In this case, the apparatus 1 of the present invention can receive data relating to mass spectrometry results, the data being input by a user or being read out from a recording medium such as CD, via an input unit 30.

Further, the apparatus 1 of the present invention may be connected to a network such as LAN or Internet. In this case, for example, a user may also operate the apparatus 1 of the present invention using a computer connected via a network to the apparatus 1. Further, a part of the structure of the apparatus 1 of the present invention may be connected via a network. For example, the apparatus 1 of the present invention may also be constructed by connecting a user computer serving as the input unit 30 and the output unit 40 to a server computer serving as the control unit 10 and the memory unit 20 via a network. Further, the control unit 10 and the mass spectrometry unit 50 in the apparatus 1 of the present invention may be connected to each other via a network.

## Claims

1. An apparatus for analysis of a nucleic acid, comprising:
a first processing unit for creating, based on a total base composition of a nucleic acid, a plurality of base composition sets each consisting of a set of partial base compositions, creating a hierarchical structure in which the base composition sets are hierarchized in ascending order of a number of bases in a partial base composition at at least one terminal position thereof, and further creating connection relations between each of the base composition sets, and another base composition set having, at the terminal position, a partial base composition obtained by adding one base to a partial base composition at the terminal position of each of the base composition sets;
a second processing unit for calculating, for each of the partial base compositions, a predicted mass value in mass spectrometry of a corresponding product ion, and imparting a weight to each of the base composition sets based on a comparison between the predicted mass value and an actual mass value actually obtained in mass spectrometry of a product ion derived from the nucleic acid; and
a third processing unit for performing selection processing for selecting one of base composition sets belonging to each hierarchy while following the connection relations from an outermost hierarchy to which the base composition sets each having a smallest number of bases in a partial base composition at the terminal position belong in the hierarchical structure, based on a weight imparted to each of the base composition sets, to thereby determine a base sequence sequentially from at least one terminus corresponding to the terminal position of the nucleic acid.

2. An apparatus for analysis of a nucleic acid according to claim 1, wherein:
the first processing unit hierarchizes the base composition sets in ascending order of a number of bases in a partial base composition at one terminal position of a 5'-terminal position and a 3'-terminal position from one outermost hierarchy in the hierarchical structure, and in ascending order of a number of bases in a partial base composition at the other terminal position of a 5'-terminal position and a 3'-terminal position from the other outermost hierarchy; and
the third processing unit performs the selection processing while following the connection relations from both outermost hierarchies of the one outermost hierarchy and the other outermost hierarchy in the hierarchical structure to internal hierarchies, to thereby determine a base sequence sequentially from both termini of a 5'-terminus and a 3'-terminus of the nucleic acid.

3. An apparatus for analysis of a nucleic acid according to claim 1 or 2, wherein:
the second processing unit imparts a weight to each of the connection relations based on a weight imparted to a pair of the base composition sets to be connected with each of the connection relations; and
the third processing unit performs the selection processing based on a weight imparted to each of the base composition sets and a weight imparted to each of the connection relations.

4. An apparatus for analysis of a nucleic acid according to any one of claims 1 to 3, wherein:
the first processing unit creates a directed graph having hierarchized nodes corresponding to each of the base composition sets, and edges for connecting the nodes in a corresponding manner to each of the connection relations; and
the third processing unit performs the selection processing by searching an optimum route from a terminus on a side of a hierarchy to which the base composition sets each having a smallest number of bases in a partial base composition at the terminal position belong in the directed graph, based on a weight imparted to each of the base composition sets.

5. An apparatus for analysis of a nucleic acid according to claim 4, wherein
the third processing unit performs the selection processing by applying dynamic programming.

6. An apparatus for analysis of a nucleic acid according to claim 5, wherein:
the first processing unit hierarchizes the nodes in ascending order of a number of bases in a partial base composition at one terminal position of a 5'-terminal position and a 3'-terminal position of the corresponding base composition sets from one terminus in the directed graph, and in ascending order of a number of bases in a partial base composition at the other terminal position of a 5'-terminal position and a 3'-terminal position of the corresponding base composition sets from the other terminus of the directed graph; and
the third processing unit performs the selection processing while following the edges by applying dynamic programming inward from both termini of the one terminus and the other terminus in the directed graph, to thereby determine a base sequence sequentially from both termini of a 5'-terminus and a 3'-terminus of the nucleic acid.

7. An apparatus for analysis of a nucleic acid according to any one of claims 1 to 6, wherein
the second processing unit creates, for each of the partial base compositions, a first predicted mass value to be predicted when an assumption is made that no modified base is contained in the corresponding product ion, and a second predicted mass value to be predicted when an assumption is made that a modified base is contained in the corresponding product ion in mass spectrometry of the corresponding product ion, and imparts a weight to each of the partial base compositions based on a comparison of each of the first predicted mass value and the second predicted mass value with the actual mass value.

8. An apparatus for analysis of a nucleic acid according to claim 7, wherein:
the second processing unit judges whether or not the modified base is contained in the corresponding product ion for each of the partial base compositions, based on a comparison of each of the first predicted mass value and the second predicted mass value with the actual mass value, and holds each of the partial base compositions linked to modification information relating to the modified base, provided that the second processing unit judges that the modified base is contained in the corresponding product ion; and
the third processing unit determines a position of a modified base contained in the nucleic acid based on judgment results of whether or not a position of a partial base composition linked to the modification information moves in the selected base composition set between adjacent hierarchies in the hierarchical structure.

9. A method for analysis of a nucleic acid, comprising:
a first step for creating, based on a total base composition of a nucleic acid, a plurality of base composition sets each consisting of a set of partial base compositions, creating a hierarchical structure in which the base composition sets are hierarchized in ascending order of a number of bases in a partial base composition at at least one terminal position thereof, and further creating connection relations between each of the base composition sets, and another base composition set having, at the terminal position, a partial base composition obtained by adding one base to a partial base composition at the terminal position of each of the base composition sets;
a second step for calculating, for each of the partial base compositions, a predicted mass value in mass spectrometry of a corresponding product ion, and imparting a weight to each of the base composition sets based on a comparison between the predicted mass value and an actual mass value actually obtained in mass spectrometry of a product ion derived from the nucleic acid; and
a third step for performing selection processing for selecting one of base composition sets belonging to each hierarchy while following the connection relations from an outermost hierarchy to which the base composition sets each having a smallest number of bases in a partial base composition at the terminal position belong in the hierarchical structure, based on a weight imparted to each of the base composition sets, to thereby determine a base sequence sequentially from at least one terminus corresponding to the terminal position of the nucleic acid.

10. A program for analysis of a nucleic acid, wherein the program causes a computer to carry out:
a first procedure for creating, based on a total base composition of a nucleic acid, a plurality of base composition sets each consisting of a set of partial base compositions, creating a hierarchical structure in which the base composition sets are hierarchized in ascending order of a number of bases in a partial base composition at at least one terminal position thereof, and further creating connection relations between each of the base composition sets, and another base composition set having, at the terminal position, a partial base composition obtained by adding one base to a partial base composition at the terminal position of each of the base composition sets;
a second procedure for calculating, for each of the partial base compositions, a predicted mass value in mass spectrometry of a corresponding product ion, and imparting a weight to each of the base composition sets based on a comparison between the predicted mass value and an actual mass value actually obtained in mass spectrometry of a product ion derived from the nucleic acid; and
a third procedure for performing selection processing for selecting one of base composition sets belonging to each hierarchy while following the connection relations from an outermost hierarchy to which the base composition sets each having a smallest number of bases in a partial base composition at the terminal position belong in the hierarchical structure, based on a weight imparted to each of the base composition sets, to thereby determine a base sequence sequentially from at least one terminus corresponding to the terminal position of the nucleic acid.


SEQUENCE LISTING

<110> The University of Tokyo, Japan Biological Informatics Consortium

<120> Device, Method, Program for Analysis of Nucleic Acid

<130> CA-0041

<160> 3

<170> PatentIn version 3.1

<210> 1
<211> 10
<212> RNA
<213> Escherichia coli

<400> 1
ucuccucaug 10

<210> 2
<211> 23
<212> RNA
<213> Artificial

<220>
<223> Artificially synthesized mmu-miR-122a

<400> 2
uggaguguga caaugguguu ugu 23

<210> 3
<211> 11
<212> RNA
<213> human

<400> 3
ucaccccauu g 11